# EUROPÄISCHE PATENTANMELDUNG

(11) **EP 1 891 936 A1**
(43) Veröffentlichungstag der Anmeldung: **27.02.2008**
(21) Anmeldenummer: 06017263.2
(22) Anmeldetag: 18.08.2006
(51) Int. Cl.: A61K 9/00, A61K 9/28, A61K 9/50

(54) **Gut schluckbare pharmazeutische Zusammensetzung aus einem oder mehreren wirkstoffhaltigen Teilchen ohne unangenehmes Mundgefühl**

(71) Anmelder: Losan Pharma GmbH, 79395 Neuenburg (DE)
(72) Erfinder: Gruber, Peter, 79249 Merzhausen (DE); Kraahs, Peter, 81825 München (DE)
(74) Vertreter: Best, Michael

(57) **Zusammenfassung**

Die Erfindung betrifft eine besonders für kleine Kinder sehr gut schluckbare pharmazeutische Zusammensetzung zur direkten oralen Verabreichung, enthaltend mindestens eine pharmazeutisch aktive Verbindung. Die pharmazeutische Zusammensetzung liegt in Form von einem oder mehreren Teilchen vor. Die Teilchen bestehen aus einem wirkstoffhaltigen Kern, auf dem ein oder mehrere Überzüge aufgebracht sind. Die pharmazeutische Zusammensetzung wird bevorzugt in Kombination mit einem Pulver und/oder Granulat verabreicht, welches bei Applikation auf die Zunge spontan zur Bildung von zusätzlichem Speichel führt. Die überzogenen Teilchen bilden innerhalb weniger Sekunden mit dem Speichel eine im Mund angenehm empfundene, weiche, glatte, aber mechanisch stabile Oberfläche, wodurch sie mit dem zusätzlich gebildeten Speichel leicht, nahezu quantitativ geschluckt werden können.

## Beschreibung

Die Erfindung betrifft eine besonders für kleine Kinder sehr gut schluckbare pharmazeutische Zusammensetzung zur direkten oralen Verabreichung, enthaltend mindestens eine pharmazeutisch aktive Verbindung. Die pharmazeutische Zusammensetzung liegt in Form von einem oder mehreren Teilchen vor. Die Teilchen bestehen aus einem wirkstoffhaltigen Kern, auf dem ein oder mehrere Überzüge aufgebracht sind. Die pharmazeutische Zusammensetzung wird bevorzugt in Kombination mit einem Pulver und/oder Granulat verabreicht, welches bei Applikation auf die Zunge spontan zur Bildung von zusätzlichem Speichel führt. Die überzogenen Teilchen bilden innerhalb weniger Sekunden mit dem Speichel eine im Mund angenehm empfundene, weiche, glatte, aber mechanisch stabile Oberfläche, wodurch sie mit dem zusätzlich gebildeten Speichel leicht, nahezu quantitativ geschluckt werden können.

Die orale Einnahme fester Arzneiformen (insbesondere von Tabletten oder Kapseln) ist bei Kindern und älteren Menschen mit zahlreichen Problemen behaftet. Kinder können erst im Alter von 6-8 Jahren zuverlässig feste Arzneiformen schlucken, wobei große Einschränkungen bezüglich der Größe der Arzneiformen bestehen. Auch alte Menschen leiden sehr häufig unter Schluckbeschwerden bedingt durch eine im Alter auftretende Verringerung der Speichelbildung wodurch die festen Arzneiformen nur mit Mühe geschluckt werden können. Die oft über Jahre notwendige, regelmäßige Einnahme stößt auf Widerwillen, was sehr häufig zu einer unregelmäßigen Einnahme führt.

Um die genannten Probleme zu überwinden, sind bereits zahlreiche Alternativen auf dem Markt. Aber auch Kautabletten, Lutschtabletten und orodispersible Tabletten stellen gerade für kleine Kinder keine echte Alternative dar. Auch ältere Menschen lehnen wegen Problemen mit den Zähnen häufig Kautabletten ab, und aufgrund der verminderten Speichelbildung sind Lutschtabletten und orodispersible Tabletten ebenfalls nicht überzeugend. Außerdem müssen die sehr häufig bitter schmeckenden Wirkstoffe vor ihrer Verarbeitung in Kautabletten, Lutschtabletten oder orodispersiblen Tabletten erst in einem teuren und komplizierten Arbeitsschritt in kleine speichelresistente Teilchen überführt werden, wobei häufig die Geschmacksmaskierung durch die anschließend notwendige Verpressung zu Tabletten mehr oder weniger zerstört wird und der bittere Geschmack wiederum zur Ablehnung dieser Arzneiformen führt. Noch problematischer ist die Verarbeitung von freigabegesteuerten magensaftresistenten oder retardierten Teilchen in die genannten Arzneiformen. Auch hier tritt häufig bei der anschließenden Tablettierung eine zumindest teilweise Zerstörung der Coatingschicht ein. So versucht man durch eine große Menge an leicht tablettierbaren Hilfsstoffen die speichelresistenten Teilchen zu schützen, was jedoch wiederum die Kosten zur Herstellung der genannten Tabletten erhöht und die Tablettengröße unnötig vergrößert.

Deshalb sind auch Granulate und Pulver auf dem Markt, die vor der Einnahme in der Regel mit einer bestimmten Menge Wasser erst in eine Suspension überführt werden müssen. Derartige Suspensionen müssen in den meisten Fällen konserviert werden, müssen oft im Kühlschrank aufbewahrt werden und führen grundsätzlich zu hohen Produktkosten. Bei der Bereitung der Suspension treten häufig Fehler auf. Durch Schaumbildung oder ungenügendes Schütteln vor der Applikation kann es zu problematischen Dosierungsfehlern kommen, und insbesondere ältere Menschen kommen mit dieser Arzneiform nicht zurecht.

Bei Kleinkindern oder auch Erwachsenen mit Schluckproblemen versucht man auch häufig auf einen Sirup auszuweichen. In vielen Fällen kommt es aber zu erheblichen Problemen aufgrund der Bitterkeit des Wirkstoffes. In allen Fällen müssen Sirupe durch Konservierungsmittel geschützt werden, die grundsätzlich ein Allergierisiko besitzen. Bei vielen Wirkstoffen treten auch durch die Verarbeitung zu einem wässrigen Sirup erhebliche Stabilitätsprobleme auf.

In der US-A-4,882,169 werden überzogene Pellets mit einem Durchmesser von 0,2-3 mm vorgeschlagen, die in Wasser eine homogene Dispersion bilden sollen. Die Partikel enthalten mindestens eine pharmazeutisch wirksame Substanz, gegebenenfalls einen oder mehrere freigabesteuernde oder geschmacksmaskierende Überzüge und eine quellbare äußere Schicht. Letztere enthält ein quellbares Polymer, vorzugsweise Guar, und ein Bindemittel. Die überzogenen Pellets werden zusammen mit Guargummi-Granulat und Aromastoffen in Sachets gefüllt, aus denen sie entnommen und in Wasser dispergiert werden.

In der US-A-5,288,500 wird vorgeschlagen, eine Vielzahl wirkstoffhaltiger Teilchen mit einem Durchmesser von 0,05 bis 7 mm mit einem Gelier- oder Quellmittel zu kombinieren. Letzteres kann im Gemisch mit den wirkstoffhaltigen Teilchen vorliegen, in einem Überzug enthalten sein, den wirkstoffhaltigen Teilchen vor dem Zumischen zu einem wässrigen Träger zugesetzt werden oder in einem wässrigen Träger, dem die wirkstoffhaltigen Teilchen zugemischt werden, dispergiert sein. Die vorgeschlagene Formulierung wird in einem wässrigen Träger dispergiert. Als Gelier- oder Quellmittel für die in US-A-5,288,500 vorgeschlagenen Formulierungen werden insbesondere hydrophile Polymere angegeben, die in wässriger Umgebung kolloide Dispersionen, Sole oder Suspensionen bilden. Die Polymere werden in einer Menge eingesetzt, die ausreicht, dass die Dispergierung in einem wässrigen Träger nicht beeinträchtigt wird. Gewünschtenfalls kann die Viskosität in unmittelbarer Nachbarschaft der dispergierten Teilchen durch Salzbildung, Chelatbildung, Veränderung der Polarität und dergleichen beeinflusst werden. Die Komponenten der Formulierung können bis zur Verabreichung getrennt gehalten oder zusammen in Sachets abgefüllt oder zu Tabletten oder Kapseln verarbeitet werden.

Die in US-A-4,882,169 und US-A-5,228,500 beschriebenen Formulierungen werden zusammen mit einem wässrigen Träger eingenommen, in welchen sie vor der Verabreichung dispergiert werden müssen. Hierbei zerfallen die Formulierungen in die Einzelteilchen, womit eine quantitative Einnahme in der Regel nicht gewährleistet ist. Generell stellt nämlich das Trinken einer Teilchensuspension oder -dispersion ein echtes Problem dar, weil nach dem Austrinken der Lösung in der Regel zumindest ein Teil der Teilchen am Boden des Gefäßes zurückbleibt und nur mühsam in den Mund überführt werden kann. Es ist auch im hohen Maße unsicher, ob der Patient dies überhaupt versucht. Nur durch sehr schnelles Trinken der Teilchensuspension nach dem Umrühren kann man eine Sedimentation der Partikel teilweise verhindern. Eine solche Arzneiform, die schnell getrunken werden muss ist insbesondere für kleine Kinder und ältere Patienten ungeeignet.

Formulierungen entsprechend der beiden genannten Patente sind mit dem Nachteil behaftet, dass sie eine quantitative Einnahme in der vorgesehenen Dosis in der Regel nicht gewährleisten und dass sie erst in einem wässrigen Träger vordispergiert werden müssen d.h. sauberes Trinkwasser und ein geeignetes Gefäß müssen zur Verfügung stehen, was z.B. auf Reisen die Einnahme erschwert.

Ein verbessertes Mittel wird in der WO 98/06385 offenbart. Die Druckschrift beschreibt eine pharmazeutische Zusammensetzung in Form von Teilchen, die direkt auch ohne Flüssigkeit eingenommen werden kann. Die pharmazeutische Zusammensetzung ist zur oralen Verabreichung und enthält ein oder mehrere überzogene Wirkstoffteilchen mit einem aus einem oder mehreren Schichten bestehenden Überzug, wobei die Zusammensetzung des Überzugs dadurch gekennzeichnet ist, dass
a) die Überzugsschicht bzw. die Überzugsschichten mindestens ein hydratisierbares Polymer enthalten, das bei Kontakt mit Speichel oder Wasser eine zusammenhängende, formbare, viskose klebende Teilchenmasse bildet, die den Austritt von wirkstoffhaltigen Teilchen aus dieser Masse und die Freisetzung von Wirkstoff in die Mundhöhle verhindert und
b) die äußerste Überzugsschicht eine wirksame Menge mindestens eines speichelflussfördernden Mittels enthält.

Der Nachteil dieser Erfindung besteht darin, dass es für kleine Kinder und auch ältere Menschen schwierig ist, die auf die Zunge geschütteten Teilchen solange zusammenzuhalten, bis gebildeter Speichel die einzelnen Teilchen zu einer Teilchenmasse verbindet. Gerade Kinder verteilen die mit dem Polymer überzogenen Teilchen im Mund und die entstehende Speichelbildung ist zu gering, als dass die an der Oberfläche klebenden Teilchen ohne die zu Hilfenahme von Wasser geschluckt werden können. Da die in den Mund geschütteten Teilchen häufig nicht oder nur unvollständig zu einer Teilchenmasse zusammenkleben, ist auch die Wirkstofffreisetzung aus den Teilchen erhöht, so dass nur noch ein ungenügender Schutz gegen unangenehm bitter schmeckenden Wirkstoffen besteht. Dieser kann aus den einzelnen Teilchen viel besser diffundieren als aus einem zusammengeklebten Teilchenklumpen mit sehr stark reduzierter Wirkstoff abgebender Oberfläche. Es ist gerade das Ziel dieser Entwicklung des Standes der Technik, nur eine solche Menge an Speichel entstehen zu lassen, die ausreicht, dass die Polymere an der Teilchenoberfläche klebrig werden und so zu einem Klumpen zusammenkleben. Es ist nicht Ziel der Entwicklung, dass unmittelbar nach der oralen Einnahme spontan ein größerer Speichelfluss entsteht, mit dem die geschmacksgeschützten Partikel leicht geschluckt werden können. Nur wenn die auf die auf Zungenmitte geschütteten Partikel geschickt ca. 30-60 sec gegen den Gaumen gedrückt werden, verkleben sie unter Bildung eines zusammenhängenden, plastischen, viskosen Teilchenbreis, der relativ einfach geschluckt werden kann. Außerdem hat es sich gezeigt, dass ein nahezu quantitatives Zusammenkleben der einzelnen Teilchen nie erreicht wird und einzelne Teilchen in die Mundhöhle, z.B. zwischen die Zähne diffundieren, was nachteilig ist.

Gerade bei Kindern ist es auch von sehr großer Wichtigkeit, dass bei Kontakt mit Speichel sich rasch eine mechanisch stabile, weiche Teilchenoberfläche bildet, die auch durch Bewegung mit der Zunge nicht entfernbar ist und den Teilchen ein angenehmes Mundgefühl verleiht. Grundsätzlich werden harte Granulatkörner, gröbere Kristalle, aber auch Pellets in Folge ihrer rauen Oberfläche im Mund als unangenehme Fremdkörper empfunden. Dies führt insbesondere bei kleinen Kindern dazu, dass die Einnahme bzw. das Schlucken dieser Teilchen verweigert wird bzw. die Kinder versuchen, die Teilchen auszuspucken. Deshalb ist es wichtig, dass innerhalb von Sekunden nach der Einnahme auf den genannten Teilchen sich eine weiche aber mechanisch stabile Oberfläche bildet. Gerade für Kinder ist es schwierig, die Wirkstoff enthaltenden Teilchen rasch und quantitativ zu schlucken. Sie neigen eher dazu, die Teilchen in der gesamten Mundhöhle zu verteilen. Außerdem üben sie durch unkontrollierte Bewegungen mit der Zunge einen starken mechanischen Stress auf die Teilchenoberfläche aus, so dass ein auf der Oberfläche befindliches gequollenes, nicht fest fixiertes Polymer sehr leicht abgerieben wird und die raue unangenehm empfundene Partikeloberfläche wieder auftritt.

Damit gerade kleine Kinder zwanglos eine pharmazeutische Zusammensetzung in Form von Teilchen schlucken, ist es sehr wichtig, dass diese Teilchen kein sandartiges, hartes, griesiges, ungewohntes Mundgefühl verursachen, sondern eine rasch sich bildende, mechanisch stabile, weiche, nicht klebende Oberfläche besitzen.

Aufgabe der Erfindung ist es, eine pharmazeutische Zusammensetzung zur Verfügung zu stellen, die die vorstehenden Probleme nicht zeigt. Die pharmazeutische Zusammensetzung soll auch von kleinen Kindern und alten Menschen leicht quantitativ eingenommen werden können, ohne dass ein unangenehmes (Mund)-Gefühl bei der Einnahme entsteht. In einer bevorzugten Ausführungsform sollte die pharmazeutische Zusammensetzung auch ohne Wasser leicht eingenommen werden können.

Diese Aufgabe wird durch den Gegenstand der Patentansprüche gelöst. Die Erfindung stellt damit eine pharmazeutische Zusammensetzung zur oralen Verabreichung in Form von zumindest einem wirkstoffhaltigen Teilchen zur Verfügung, umfassend
a) einen Kern, der den Wirkstoff des wirkstoffhaltigen Teilchens und gegebenenfalls geeignete Hilfsstoffe enthält und
b) eine oder mehrere Beschichtungen, die auf dem Kern aufgebracht sind, wobei die eine Beschichtung oder die äußerste der mehreren Beschichtungen
   (i) mindestens ein mit Wasser gelbildendes Polymer,
   (ii) mindestens ein mit Wasser nicht-gelbildendes Polymer,
   (iii) mindestens eine Verbindung, die Kohlendioxid freisetzen kann, sowie
   (iv) gegebenenfalls weitere Hilfsmittel
   enthält.

In einer bevorzugten Ausführungsform liegt die erfindungsgemäße pharmazeutische Zusammensetzung in Kombination mit einer oder mehreren den Speichelfluss fördernden Verbindungen vor. Diese Ausführungsform ist besonders dann bevorzugt, wenn die pharmazeutische Zusammensetzung ohne Hilfsmittel wie Wasser oder andere Nahrungsmittel verabreicht werden soll.

Die Erfindung beruht auf der überraschenden Erkenntnis, dass eine sich rasch ausbildende, mechanisch stabile und trotzdem weiche aber nicht klebrige Oberfläche bei Wirkstoffteilchen erreicht werden kann, wenn die Wirkstoffteilchen mit einer Beschichtung versehen sind, in der ein speichelresistentes, d.h. im Speichel (bzw. in Wasser) nicht gelbildendes Polymer mit einem im Speichel (bzw. Wasser) gelbildenden (und damit rasch quellenden) Polymer kombiniert wird und bei Kontakt mit Speichel oder Wasser in der Beschichtung Kohlendioxid entsteht. Ohne dass die Anmelder durch eine Theorie gebunden sein wollen, wird angenommen, dass das Kohlendioxid in Form von Kohlendioxidmikrobläschen entsteht, die die Beschichtung "aufblähen" und dadurch die vorteilhafte Oberfläche erzeugen.

Unter einem mit Wasser gelbildenden Polymer wird ein Polymer verstanden, das in Wasser in 2%iger Lösung oder kolloidaler Dispersion bei pH 7,0 ein Gel ausbildet. Unter einem mit Wasser nicht-gelbildenden Polymer wird ein Polymer verstanden, das in Wasser bei pH 7,0 kein Gel ausbildet und unlöslich ist.

Zur Definition eines Gels wird auf das Standardwerk Römpp, Lexikon der Chemie, 10. Auflage, Thieme Verlag, Stuttgart, verwiesen. Der pH-Wert von 7,0 wird, falls erforderlich, durch Zusatz einer Säure wie HCl (bei alkalischen Polymeren) oder einer Base wie NaOH (bei sauren Polymeren) eingestellt.

Erfindungsgemäß kann ein gelbildendes bzw. nicht-gelbildendes Polymer alternativ auch über die Viskosität definiert werden. Danach handelt es sich bei einem gelbildenden Polymer um ein Polymer, das in 2%iger (Gew./Gew.) wässriger Dispersion oder Lösung bei einem pH-Wert von 7,0 eine Viskosität von mindestens 1000 mPa·s, bevorzugt mindestens 2000 mPa·s, stärker bevorzugt mindestens 5000 mPa·s und insbesondere mindestens 10.000 mPa·s aufweist. Die pH-Werteinstellung auf pH 7,0 erfolgt gegebenenfalls wie vorstehend beschrieben mit einer Säure oder Base.

Dementsprechend handelt es sich bei einem nicht-gelbildenden Polymer um ein Polymer, das in 2%iger (Gew./Gew.) wässriger Dispersion bei einem pH-Wert von 7,0 eine Viskosität von nicht mehr als 200 mPa·s, bevorzugt nicht mehr als 100 mPa·s und insbesondere nicht mehr als 50 mPa·s aufweist. Die pH-Werteinstellung auf pH 7,0 erfolgt gegebenenfalls wie vorstehend beschrieben mit einer Säure oder Base.

Insbesondere werden erfindungsgemäß unter einem gelbildenden Polymer diejenigen Polymere verstanden, die in dieser Beschreibung ausdrücklich als gelbildende Polymere oder als Bestandteil b)(i) genannt werden, und unter einem nicht-gelbildenden Polymer werden erfindungsgemäß insbesondere diejenigen Polymere verstanden, die in dieser Beschreibung ausdrücklich als nicht-gelbidende Polymere oder als Bestandteil b)(ii) genannt werden.

Das Ausmaß der Gelbildung kann über eine Viskositätsmessung bestimmt werden. Die folgenden Ausführungen zur Viskosität gelten, sofern nichts anderes angegeben oder offensichtlich ist, für alle im Rahmen dieser Anmeldung angegebenen Viskositätswerte.

Für eine Reihe von Polymeren sind Vorschriften für die Viskositätsbestimmung in der Ph. Eur. 5. Ausgabe, Grundwerk 2005 beschrieben. Für diese Polymere wird die Viskosität bestimmt, wie es dort verlangt wird, allerdings in einer 2%igen Lösung oder Dispersion bei einem pH-Wert von 7,0.

Sollte für ein Polymer in der Ph. Eur. 5 Ausgabe, Grundwerk 2005 keine Vorschrift für die Bestimmung der Viskosität angegeben sein, oder ist die angegebene Vorschrift nicht ausreichend klar oder durchführbar, wird die Viskosität wie folgt bestimmt:

Eine 2,00 g getrockneter Substanz entsprechende Menge wird in 50 g Wasser R unter Rühren suspendiert. Die Suspension wird mit Wasser R und, zur Einstellung des pH-Werts auf 7,0, gegebenenfalls mit einer geeigneten Base (wie NaOH) oder einer geeigneten Säure (wie HCl) zu 100,0 g verdünnt und gerührt, bis die Substanz vollständig dispergiert oder gelöst ist. Die Viskosität wird mit Hilfe eines Rotationsviskosimeters bei 25 °C und einem Schergefälle von 100 s⁻¹ für Substanzen mit einer erwarteten Viskosität von höchstens 100 mPa·s, einem Schergefälle von 10 s⁻¹ für Substanzen mit einer erwarteten Viskosität zwischen 100 und 20 000 mPa·s und einem Schergefälle von 1 s⁻¹ für Substanzen mit einer erwarteten Viskosität größer als 20 000 mPa·s bestimmt. Wenn das vorgeschriebene Schergefälle nicht genau eingehalten werden kann, wird ein etwas höheres und ein etwas niedrigeres Schergefälle angewendet und anschließend interpoliert.

Falls die Bestimmung der Viskosität auf diese Methode nicht möglich ist, wird die Viskosität durch folgende Methode bestimmt:

In einem 500-ml-Becherglas werden unter Rühren mit einem leicht geneigten Propellerrührer bei einer Rotationsgeschwindigkeit von 800 U · min⁻¹ 6,0 g Substanz innerhalb von 45 bis 90 s in 250 ml einer Lösung von Kaliumchlorid R (12 g/l) eingetragen, und der pH-Wert wird, falls erforderlich, mit einer geeigneten Base (wie NaOH) oder einer geeigneten Säure (wie HCl) auf pH 7,0 eingestellt. Beim Zusatz der Substanzen dürfen keine Aggregate bestehen bleiben. Mit weiterem Wasser R werden an der Wand des Becherglases haftende Rückstände abgespült, so dass sich eine Masse von 300 g ergibt. Die Zubereitung wird 2 h lang bei einer Temperatur von 25°C mit einer Rotationsgeschwindigkeit von 800 U · min⁻¹ gerührt. Die Viskosität ist innerhalb von 15 min zu bestimmen, wobei ein mit 60 U · min⁻¹ laufendes Rotationsviskosimeter zu verwenden ist. Das Viskosimeter ist mit einer Rotationsspindel ausgestattet, deren Durchmesser 12,7 mm und deren Höhe 1,6 mm beträgt. Die Rotationsspindel ist an einem Schaft mit einem Durchmesser von 3,2 mm angebracht. Der Abstand vom oberen Teil des Zylinders zum unteren Ende des Schafts beträgt 25,4 mm, die Eintauchtiefe 50,0 mm.

Diese Vorschrift ist an die Vorschrift in der Ph. Eur. 5 Ausgabe, Grundwerk 2005 für Xanthangummi angelehnt.

Sofern nichts anderes ausdrücklich offenbart oder für einen Fachmann offensichtlich ist, wird erfindungsgemäß ein pH-Wert in einer 3%igen wässrigen Dispersion oder Lösung bei 25°C bestimmt.

Sofern nichts anderes ausdrücklich offenbart oder für den Fachmann offensichtlich ist, beziehen sich Teile und Verhältnisse immer auf das Gewicht.

Die erfindungsgemäße pharmazeutische Zusammensetzung zur oralen Verabreichung liegt in Form von zumindest einem wirkstoffhaltigen Teilchen vor. In der Regel besteht die pharmazeutische Zusammensetzung jedoch aus mehreren wirkstoffhaltigen Teilchen. Die Größe der wirkstoffhaltigen Teilchen, die die erfindungsgemäße pharmazeutische Zusammensetzung zur oralen Verabreichung ausmachen, ist nicht besonders eingeschränkt. Handelt es sich bei der erfindungsgemäßen pharmazeutischen Zusammensetzung um ein einziges Teilchen (z.B. eine Tablette), kann das Teilchen eine Größe von bis zu etwa 15 mm, bevorzugt bis zu etwa 10 mm, aufweisen. Möglich sind auch z.B. sogenannte Oblong-Tabletten, die eine Länge von bis zu 25 mm, eine Dicke von bis zu 10 mm und eine Breite von bis zu 12 mm haben. Besteht die pharmazeutische Zusammensetzung aus mehreren Teilchen, liegt die Größe der Teilchen in der Regel im Bereich von 0,05 bis etwa 7 mm, bevorzugt 0,1 bis 5 mm, stärker bevorzugt 0,2 bis 3 mm.

Ein erfindungsgemäßes wirkstoffhaltiges Teilchen umfasst einen Kern und eine oder mehrere Beschichtungen. Der Wirkstoff befindet sich in der Regel ausschließlich im Kern der wirkstoffhaltigen Teilchen. Geeignete wirkstoffhaltige Kerne sind z.B. auf klassisch hergestellte Weise gesiebte Granulate mit einer Korngröße 0,05 bis 1,0 mm mit rauer Oberfläche oder scharfkantige Wirkstoffkristalle in einem Korngrößenbereich von 0,05 bis 2,0 mm. Selbstverständlich stellen auch aus sogenannten Nonpareilles durch Auftragen von Wirkstoff oder Wirkstoffen hergestellte Pellets, insbesondere solche mit rauer Oberfläche, ebenso wie Pellets, die durch das bekannte Verfahren der Extrusion und Spheronisation einer wirkstoffenthaltenden plastischen Masse hergestellt wurden, geeignete wirkstoffhaltige Kerne dar. Schließlich ist die Erfindung auch besonders geeignet für Minitabletten mit 1,5 bis 3,5mm, da diese durch die Verpressung scharfe Kanten haben können, die besonders unangenehm beim Schlucken empfunden werden.

Die erfindungsgemäße Zusammensetzung eignet sich grundsätzlich zur Verabreichung beliebiger therapeutisch und/oder prophylaktisch wirksamer oral verabreichbarer Wirkstoffe (Phyto-Extrakte, Mineralien, Spurenelemente, Vitamine, Vitalstoffe), wobei diese in den Kernen, d.h. als Granulat, Pellet oder Mikrotablette in einer Größe von 0,05 bis 3,5 mm (vor der Beschichtung) vorliegen.

Bei den Wirkstoffen handelt es sich beispielsweise um Magen-/Darmmittel und verdauungsfördernde Mittel wie Loperamid, Metoclopramid, Pankreatin, Amylase, Protease, Lipase oder Sulfasalazin, Olsalazin und Mesalazin und Cimetidin, Ranitidin, Famotidin, Nizatidin und Protonenpumpeninhibitoren wie Omeprazol, Pantoprazol, Lansoprazol, Laxantien wie Bisacodyl, Natriumpicosulfat.

Geeignete Wirkstoffe sind insbesondere auch Analgetika und Antirheumatika wie Acetylsalicylsäure, Paracetamol, Ibuprofen und seine wasserlöslichen Salze des Natriums, Kaliums und des Lysins, Tramadol, Acemetacin, Naproxen, Naproxen-Natrium, Diclofenac, Ketoprofen, Piroxicam, Indometacin wie auch Antiallergika wie Astemizol, Ketotifen, Cetirizin, Loratadin und Fexofenadin, β-Rezeptorenblocker, Calciumantagonisten und ACE-Hemmer, Koronarmittel, Antiparkinsonmittel und Sedativa wie Nitrazepam, Oxazepam, Docusate-Natrium, Zolpidem. Weitere geeignete Wirkstoffe sind Antibiotika wie Ciprofloxacin, Norfloxacin, Ofloxacin, Nalidixinsäure, Cinoxacin, Pefloxacin, Phenoxymethyl-Penicillin, Amoxicillin und weitere Penicilline mit einer Penam-Struktur, Cephalosporine mit einer Cephem-Struktur wie Cefaclor, Cefadroxil, Cefalexin, Cefpodoxim, Ceftibuten, Cefuroxim und Cefetamet, Clavulansäure in Kombination mit Amoxicillin, Tetracyclin, Makrolid-Antibiotika wie Erythromycin und seine Ester, Spiramycin und Josamycin, Colistin und Polymycin B, Nitrofurane wie Nitrofurantoin, Nitroimidazole wie Metronidazol und Wirkstoffe wie Clarithromycin, Azithromycin, Griseofulvin und Sulfamethoxazol in Kombination mit Trimethoprim. Ferner eignen sich die erfindungsgemäße Zusammensetzung auch zur Verabreichung von Mineralsalzen des Calciums, des Magnesiums, des Zinks, des Eisens und von Spurenelementen wie z.B. Chrom, Mangan, Selen usw. und insbesondere von allen Vitaminen. Gewünschtenfalls kann die Zusammensetzung Kombinationen von Vitaminen und/oder Mineralien und/oder Spurenelementen mit/ohne Vitalstoffen (Carnitin, Soya-Isoflavone, Phytosterole) enthalten. Besonders geeignet sind insbesondere Wirkstoffe, die in der Pädiatrie eingesetzt werden wie die Antitussiva and Expectorantia Guaifenesin, Ambroxol, Bromhexin, Acetylcystein, Codein, Theophyllin und z.B. Kombinationen des Guaifenesins mit Dextromethorphan-Hydrobromid, Phenylephrin-HCl und Diphenhydramin-HCl. Dabei können die erfindungsgemäßen wirkstoffhaltigen Partikel z.B. das Guaifenesin kombiniert mit Phenylephrin oder Dextromethorphan-HBr enthalten bzw. können z.B. erfindungsgemäße hergestellte Wirkstoffpartikel des Guaifenesins und des Dextromethorphans bzw. des Phenylephrins miteinander und gegebenenfalls mit der speichelbildenden Komponente gemischt werden. Bevorzugt sind auch Präparate, die als pharmazeutischen Wirkstoff Steroide wie Prednisolon und seine Salze enthalten.

Grundsätzlich sind insbesondere Wirkstoffe geeignet, die einen unangenehmen und/oder bitteren Geschmack aufweisen wie Guaifenesin, Prednisolon, Codein und Koffein und Wirkstoffe mit einer hohen Dosismenge und gegebenenfalls zusätzlich unangenehmem Geschmack wie 5-Aminosalicylsäure (Einzeldosis bis zu 1,5 g), Gabapentin (Einzeldosis bis 1,5 g), Ibuprofen und seine wasserlöslichen Salze (Einzeldosis bis 800 mg Ibuprofen) und Paracetamol (Einzeldosis bis 1,0 g). Aber auch Kombinationen von Paracetamol und/oder Acetylsalicylsäure und/oder Koffein und/oder Vitamin C sind geeignete WirkstoffKombinationen. Besonders bevorzugt ist erfindungsgemäß der Wirkstoff Guaifenesin, der Wirkstoff Dextromethorphan (oder ein Salz davon), ein Gemisch aus Guaifenesin und Dextromethorphan (oder einem Salz davon), ein Gemisch aus Guaifenesin und Phenylephrin (oder einem Salz davon) oder Prednisolon. Prednisolon kann gegebenenfalls auch in Form eines Esters oder Salzes vorliegen, wie als Prednisolonacetat oder Prednisolonsulfat-Na.

Wenn im Rahmen dieser Anmeldung von einem Wirkstoff gesprochen wird, ist hierunter ein einzelner Wirkstoff oder ein Gemisch von mehreren Wirkstoffen zu verstehen.

Neben dem Wirkstoff kann der Kern der erfindungsgemäßen pharmazeutischen Zusammensetzung noch übliche pharmazeutische Hilfsstoffe enthalten, die dem Fachmann bekannt sind, insbesondere die üblicherweise zur Herstellung von Granulatpellets oder Mikrotabletten verwendeten Hilfsstoffe. Zur Herstellung der erfindungsgemäßen wirkstoffhaltigen Teilchen kann auf die übliche Standardliteratur zur pharmazeutischen Technologie verwiesen werden, z.B. auf "W.A. Ritschel, A. Bauer-Brandl, Die Tablette, Editio-Cantor-Verl., 2002".

Erfindungsgemäß wesentlich ist, dass der Kern der erfindungsgemäßen pharmazeutischen Zusammensetzung eine oder mehrere Beschichtungen aufweist. In dem Fall, dass mehrere Beschichtungen vorhanden sind, sind diese Beschichtungen in der Regel übereinander aufgebracht, und die äußerste dieser Beschichtungen ist erfindungsgemäß besonders wesentlich. Diese äußerste der Beschichtungen bzw. in dem Fall, dass nur eine Beschichtung aufgebracht ist, die eine Beschichtung, wird im Folgenden als erfindungsgemäße Beschichtung bezeichnet.

Die erfindungsgemäße Beschichtung enthält zumindest drei Bestandteile, die voneinander verschieden sind. Einer der Bestandteile ist ein mit Wasser gelbildendes Polymer, das bevorzugt in Wasser bei einem pH von 3,5 oder weniger (und damit auch im Magensaft) löslich ist, oder eine Dispersion bildet. Dieses Polymer quillt infolge der raschen Aufnahme von Wasser oder Speichel. Der zweite Bestandteil ist ein mit Wasser nicht-gelbildendes Polymer, das daher bei Kontakt mit Wasser oder Speichel praktisch nicht quillt. Es ist bei einem pH von 7,0 und damit auch im Speichel im wesentichen unlöslich. Dieses mit Wasser nicht-gelbildende Polymer kann im Magensaft löslich sein oder kann auch im Magensaft unlöslich sein. Bevorzugt handelt es sich hierbei um ein Polymer, das in Wasser bei einem pH von 5 oder weniger (und damit auch im Magensaft) löslich ist, oder eine Dispersion bildet, die eine Viskosität von 100 mPa·s oder weniger und bevorzugt von 50 mPa·s oder weniger, insbesondere von 10 mPa·s oder weniger, aufweist (2% Gew./Gew. Dispersion).

Der dritte wesentliche Bestandteil der erfindungsgemäßen Beschichtung ist eine Verbindung, die Kohlendioxid freisetzen kann. Erfindungsgemäß kann eine derartige Verbindung oder mehrere derartige Verbindungen in der erfindungsgemäßen Beschichtung vorhanden sein. Besonders bevorzugt handelt es sich bei diesen Verbindungen, die Kohlendioxid freisetzen können, um anorganische Carbonate oder Hydrogencarbonate, besonders bevorzugt sind Carbonate und Hydrogencarbonate der Alkalimetalle und Erdalkalimetalle, also Verbindungen der Formel MHₙCO₃, wobei M ein Alkalimetallion oder ein Erdalkalimetallion ist und n = 0 oder 1 ist, wobei n = 0 ist, wenn M ein Erdalkalimetallion ist. Am stärksten bevorzugt sind Natriumhydrogencarbonat, Kaliumhydrogencarbonat, Calciumcarbonat, Magnesiumcarbonat und Gemische aus diesen Verbindungen. Erfindungsgemäß bevorzugt ist ebenfalls Natriumglycincarbonat. Erfindungsgemäße am stärksten bevorzugt sind die Hydrogencarbonate Natriumhydrogencarbonat und Kaliumhydrogencarbonat sowie Gemische davon.

In einer weiteren Ausführungsform kann die erfindungsgemäße Beschichtung neben den basischen Kohlendioxid-freisetzenden Verbindungen noch weitere basische Hilfsstoffe enthalten, wie Trinatriumcitrat, Trinatrium-/Trikaliumcitrat oder Trinatriumphosphat. Die Kohlendioxid-freisetzenden Verbindungen, insbesondere die Hydrogencarbonate/Carbonate, Natrium-/Kaliumhydrogencarbonat, Natrium-/Kaliumcarbonat, Calciumcarbonat, Magnesiumcarbonat, Natriumglycincarbonat sowie die gegebenenfalls weiteren basischen Hilfsstoffe, die gegebenenfalls zusätzlich verwendet werden können, werden bei der Herstellung der erfindungsgemäßen Beschichtung bevorzugt in mikronisierter Form eingesetzt. Die mittlere Teilchengröße ist bevorzugt max. 0,1 mm, ganz bevorzugt 5 bis max. 50 µm.

Die anorganischen Carbonate bzw. Hydrogencarbonate setzen insbesondere dann Kohlendioxid frei, wenn sie mit sauren Verbindungen und Wasser in Kontakt kommen. Daher enthält die erfindungsgemäße Beschichtung neben der Verbindung, die Kohlendioxid freisetzen kann, bevorzugt auch noch eine Verbindung, die, wenn sie mit Wasser oder Speichel in Kontakt kommt, eine Säure zur Verfügung stellt, die die Freisetzung des Kohlendioxids begünstigt oder bewirkt. Erfindungsgemäß kann es sich bei dem mit Wasser gelbildenden Polymer oder bei dem mit Wasser nicht-gelbildenden Polymer um ein saures Polymer handeln. Es ist auch möglich, dass es sich auch bei beiden Polymeren um saure Polymere handelt. Unter einem "sauren Polymer" wird erfindungsgemäß ein Polymer verstanden, das in wässriger Lösung oder Dispersion einen pH-Wert <7 zur Verfügung stellt, bevorzugt wird hierunter ein Polymer verstanden, das in 3%iger Lösung oder Dispersion in Wasser einen pH-Wert ≤ 6, stärker bevorzugt ≤ 5, zur Verfügung stellt.

Erfindungsgemäß ebenfalls bevorzugt ist es, dass der erfindungsgemäßen Beschichtung auch noch eine saure Verbindung zugesetzt wird, wie später beschrieben. Sobald die erfindungsgemäße Beschichtung daher im Mund mit Wasser oder Speichel in Kontakt kommt, bilden sich in der erfindungsgemäßen Schicht Kohlendioxidmikrobläschen, die gemeinsam mit den weiteren Bestandteilen der erfindungsgemäßen Beschichtung dafür sorgen, dass es innerhalb von Sekunden zur Ausbildung einer weichen, im Mund angenehm empfundenen, aber mechanisch stabilen Teilchenoberfläche kommt.

Mit Wasser gelbildende Polymere sind bekannt. Wird diesen Polymeren Wasser oder Speichel zur Verfügung gestellt (was bei einer Verabreichung direkt in den Mund eines Patienten immer der Fall ist), quellen diese Polymere durch die vorhandene Wasser- oder Speichelmenge zu einem Gel auf. Diese Polymere zeichnen sich daher bevorzugt auch durch ein besonders gutes Quellvermögen aus. Besonders bevorzugt handelt es sich bei diesen mit Wasser gelbildenden Polymeren um Polyacrylsäuren und Polyacrylate, vor allem solche mit einem Molekulargewicht von 400.000 bis 4 Millionen.

Ganz besonders bevorzugt handelt es sich bei den mit Wasser gelbildenden Polymeren um saure Polymere, da diese bei Kontakt mit Wasser oder Speichel direkt mit den Kohlendioxid-freisetzenden Verbindungen unter Freisetzung von Kohlendioxid reagieren können. Bevorzugt sind hierbei saure Polymere, die Carboxyl- oder Schwefelsäure-Gruppen enthalten, wie die Carbopole und Polycarbophile, aber auch Alginsäure mit einer Viskosität von > 1000 mPa·s bei einem pH-Wert von 7,0 und einem Molekulargewicht von etwa 200.000; Carragenane mit einer Viskosität von mindestens 1000 mPa·s (2%ige Lösung) und Pektine, wobei diese bei gewünschter langsamer Quellung eingesetzt werden.

Bevorzugte nicht saure Polymere gemäß Bestandteil b)(i) sind Polyethylenoxid mit einem Molekulargewicht von 4 Millionen bis 7 Millionen und einer Viskosität einer 2%igen Lösung von 1000 bis 10.000 mPa·s, Xanthan (neutralisiert auf einen pH-Wert > 6,0) mit einer Viskosität von 1000 bis 3000 mPa·s, einem pH von etwa 6 und einem Molekulargewicht von über 100.000; Natriumcarboxymethylcellulose mit einer Viskosität von 1000 bis 8000 mPa·s, vorzugsweise 2000 bis 8000 mPa·s, mit einem Polymerisationsgrad von 500 bis 2000 und einem Substitutionsgrad von höchstens 3, d.h. höchstens 3 Carboxymethylgruppen pro Anhydroglucoseeinheit; Cellulosether mit einer Viskosität von 1000 bis 10.000 mPa·s, einem Polymerisationsgrad bis 2000 und einem Substitutionsgrad von höchstens 3, wie z.B. Hydroxyethylcellulose, Hydroxypropylcellulose, Methylhydroxypropylcellulose mit einem Substitutionsgrad von 1 bis 2, vorzugsweise einem Methyloxylgehalt von 18 bis 32 % und einem Hydroxypropylgehalt von 7 bis 15%.

Am stärksten bevorzugt sind für den Bestandteil b)(i) Carbomere (Bezeichnung laut Europäischer Pharmakopoe), die unter der Bezeichnung Carbopole vertrieben werden und bei denen es sich nach Kenntnis der Erfinder um Homopolymere der Acrylsäuren, die üblicherweise mit Allylpentaerythrol quervernetzt sind, handelt, und Polycarbophil, d.h. Homopolymere der Acrylsäure, quervernetzt mit Divinylglycol. Als 1 %ige wässrige Lösung oder Dispersion haben diese Verbindungen einen pH-Wert von 2,0 bis 3,0 und können so problemlos mit Alkali-/Erdalkali-Hydrogencarbonaten/-Carbonaten reagieren. Als Bestandteil b)(i) sind erfindungsgemäß ganz besonders Polymere bevorzugt, die nicht nur mit Wasser gelbildend sind, sondern die auch im Magensaft, also bei einem pH-Wert von unter 3, noch löslich sind oder das kolloide, disperse Gel einen starken Viskositätsabbau erleidet, da dann eine besonders schnelle Freisetzung der wirkstoffhaltigen Kerne (gegebenenfalls mit weiteren Beschichtungen) im Magen gewährleistet werden kann.

Bevorzugte mit Wasser gelbildende Polymere sind erfindungsgemäß auch solche, die sauer sind und mit steigendem pH-Wert stark quellen und mit abnehmendem pH-Wert ihre Viskosität wieder abbauen. Dies sind insbesondere Polyacrylsäuren (wie das Handelsprodukt Carbopol), quervernetzte Polyacrylsäuren wie Polycarbophil und Alginsäuren. Geeignet sind aber auch typische Celluloseether wie Methylcellulose, Hydroxyethylcellulose, Hydroxypropylcellulose und Hydroxypropylmethylcellulose und ionische Polymere wie Natriumcarboxymethylcellulose, Pektin, Xanthan, Galaktomanan, Guargummi, Hydroxypropylguargummi, Natriumalginat und dergleichen.

Die mit Wasser gelbildenden Polymere weisen vorzugsweise eine mittlere Korngröße von höchstens 0,25 mm, typischerweise 0,05 bis 0,1 mm auf. Ist eine möglichst rasche und gleichmäßige Bildung einer weichen, aber mechanisch stabilen Partikeloberfläche gewünscht, wird vorzugsweise eine sehr kleine Korngröße gewählt. In der Regel werden bevorzugt gelbildende Polymere mit einer mittleren Korngröße von höchstens 50 µm, ganz bevorzugt 1 bis 25 µm eingesetzt.

Bei der Verbindung gemäß b)(ii), das heißt bei dem mit Wasser nicht-gelbildenden Polymer, handelt es sich besonders bevorzugt um ein Eudragit-Polymer, insbesondere ein Eudragit-Polymer der Type E. Zur Definition der Eudragit-Polymere kann auf "Fiedler, Lexikon der Hilfsstoffe zur Pharmazie, Kosmetik und angrenzende Gebiete, 5. Auflage, 2002" verwiesen werden. Bei den mit Wasser nicht-gelbildenden Polymeren handelt es sich damit besonders bevorzugt um ein Copolymerisat mit kationischem Charakter auf Basis von Dimethylaminoethylmethacrylat und neutralen Methacrylsäureestern, die häufig ein mittleres gewichtsgemitteltes Molekulargewicht von ca. 150.000 aufweisen. Die Verbindungen sind auch unter der chemischen Bezeichnung Poly(butyl methacrylate, (2-dimethylaminoethyl)methacrylate, methyl methacrylate) 1:2:1 bekannt. Obwohl das Polymer Eudragit E besonders bevorzugt ist, können auch andere Polymere als Bestandteil b)(ii) eingesetzt werden, wie Eudragit E30D, Eudragit RS und Eudragit RS30D, Eudragit RL und Eudragit RL30D, sowie Ethylcellulose und Celluloseacetat. Während Eudragit E bei einem pH-Wert von unter 5 und damit ebenfalls im Magensaft löslich ist, sind einige andere der vorstehenden Polymere nicht magensaftlöslich. In diesem Fall kann aber durch ein geeignetes Verhältnis zwischen dem mit Wasser gelbildenden Polymer und dem mit Wasser nicht-gelbildenden Polymer und den verwendeten Kohlendioxid-freisetzenden Mitteln erreicht werden, dass sich die Freisetzung im Magen kaum verringert. Entsprechende Verhältnisse können leicht durch Routineversuche aufgefunden werden. Eine größere Menge an gelbildendem Polymer (Bestandteil b)(i)) im Verhältnis zum eingesetzten nicht-gelbildenden Polymer (Bestandteil b)(ii)) erhöht die Freisetzung im Magensaft. Eine größere Menge an Kohlendioxid-freisetzender Verbindung (insbesondere den anorganischen Carbonaten) verursacht eine Zerstörung der erfindungsgemäßen Beschichtung, so dass sie jegliche freisetzungsverzögernde Funktion verliert. Dies geschieht unter anderem durch die Kohlendioxidentwicklung innerhalb der erfindungsgemäßen Beschichtung, wenn die Pellets in Kontakt mit dem sauren Magensaft kommen.

Die erfindungsgemäße Beschichtung kann ebenfalls noch saure Bestandteile enthalten, insbesondere wenn die in der Beschichtung verwendeten Polymere nicht ausreichend sauer sind, um bei der Verwendung eines anorganischen Carbonats eine ausreichende Kohlendioxidentwicklung zu gewährleisten. Diese Säuren können natürlich auch im Falle des Einsatzes von sauren Polymeren zusätzlich zur Verstärkung der CO₂-Bildung zugesetzt werden. Besonders geeignete Säuren, die in Einzelfällen der erfindungsgemäßen Beschichtung zugemischt werden können, sind z.B. Mononatriumhydrogencitrat, Adipinsäure, Fumarsäure und jede andere im pharmazeutischen Bereich übliche untoxische Säure wie Zitronensäure, Weinsäure aber auch Mononatriumtartrat oder z.B. Mononatriumphosphat.

Besonders bevorzugt werden die Bestandteile der erfindungsgemäßen Beschichtung so gewählt, dass eine 3%ige Dispersion der Beschichtung, das heißt eine 3%ige Dispersion aus dem mit Wasser gelbildenden Polymer, dem mit Wasser nicht-gelbildenden Polymer, der Kohlendioxid-freisetzenden Verbindung und gegebenenfalls der Säure und weiteren Hilfsmitteln mindest einen pH von 4, stärker bevorzugt mindestens einen pH von 5,5, aufweist. Hierdurch wird sichergestellt, dass sich das nicht-gelbildende Polymer im Mund nicht auflöst. Bei geringerem pH-Wert besteht darüber hinaus das Risiko, dass die mit Wasser gelbildende Polymere, die als Verdickungsmittel wirken können, häufig einen zu starken Viskositätsabfall aufweisen, was die Gelbildung der Polymere deutlich reduziert.

Erfindungsgemäß können sich zwischen dem wirkstoffhaltigen Kern der pharmazeutischen Zusammensetzung und der erfindungsgemäßen Beschichtung noch eine oder mehrere weitere Beschichtungen befinden. Insbesondere kann eine solche weitere Beschichtung, die sich zwischen dem Kern und der erfindungsgemäßen Beschichtung befindet, eine enterische oder eine Freigabe-steuernde Beschichtung sein. Derartige Beschichtungen sind im Stand der Technik bekannt, und diesbezüglich kann auf entsprechende Standardwerke der pharmazeutischen Technologie verwiesen werden.

Die Mengen der einzelnen Bestandteile der erfindungsgemäßen Beschichtung sind nicht besonders eingeschränkt und können von einem Fachmann in Abhängigkeit der konkret verwendeten Polymere b)(i) und b)(ii) und der konkret verwendeten Kohlendioxid-freisetzenden Verbindung b)(iii) durch Routineversuche ermittelt werden, wobei er sich an den erfindungsgemäßen Beispielen orientieren kann. Allerdings ist das Verhältnis zwischen Polymer b)(ii) und Polymer b)(i) bevorzugt 10:1 bis 1:5, stärker bevorzugt 3:1 bis 1:3 und ganz bevorzugt 1,5:1 bis 1:1,5. Das Verhältnis zwischen dem Polymer b)(i) und der Kohlendioxid-freisetzenden Verbindung ist bevorzugt 10:1 bis 1:5, stärker bevorzugt 2:1 bis 1:2.

Die Masse der erfindungsgemäßen Beschichtung auf den wirkstoffhaltigen, gegebenenfalls beschichteten Kernen beträgt 1 bis 40%, bevorzugt 3 bis 25%, ganz bevorzugt 6 bis 20%, bezogen auf die Gesamtmasse der erfindungsgemäßen Teichen.

Die erfindungsgemäße Beschichtung wird im Magensaft zerstört (falls alle verwendeten Polymere im Magensaft löslich sind) oder soweit aufbläht (falls z.B. das Polymer gemäß b)(ii) im Magensaft unlöslich ist), dass sie keinen Einfluss auf die Freisetzung des Wirkstoffs hat. Dies geschieht sehr schnell, bevorzugt in weniger als 30 Minuten, stärker bevorzugt in weniger als 10 Minuten, insbesondere in weniger als 5 Minuten.

In einer bevorzugten Ausführungsform ist die erfindungsgemäßen pharmazeutische Zusammensetzung zur direkten Verabreichung in den Mund eines Patienten vorgesehen, also ohne dass sie vorher in Wasser eingebracht wird. In diesem Fall ist es vorteilhaft, die erfindungsgemäße pharmazeutische Zusammensetzung zusammen mit einer oder mehreren Speichelfluss-fördernden Verbindung zu verabreichen. Die Erfindung betrifft damit auch ein Arzneimittel, das neben der erfindungsgemäßen pharmazeutischen Zusammensetzung noch mindestens eine den Speichelfluss-fördernde Verbindung enthält. Diese Verbindung ist nicht Bestandteil der pharmazeutischen Zusammensetzung (obwohl erfindungsgemäß nicht ausgeschlossen ist, dass die erfindungsgemäße Beschichtung der erfindungsgemäßen Zusammensetzung auch zusätzliche Bestandteile enthält, die den Speichelfluss fördern).

Das Arzneimittel mit der erfindungsgemäßen pharmazeutischen Zusammensetzung kann so ausgestaltet sein, dass es die erfindungsgemäße pharmazeutische Zusammensetzung und die den Speichelfluss fördernde Verbindung im Gemisch enthält. Dies ist erfindungsgemäß bevorzugt. Alternativ können in dem Arzneimittel die erfindungsgemäße pharmazeutische Zusammensetzung und die den Speichelfluss fördernde Verbindung aber auch getrennt zur gleichzeitigen Verabreichung vorliegen oder die erfindungsgemäße pharmazeutische Zusammensetzung kann mit der Anweisung versehen sein, sie gleichzeitig mit einer den Speichelfluss fördernden Verbindung einzunehmen.

Für die sofort speichelbildende Komponente eignen sich grundsätzlich beliebige pharmazeutisch annehmbare Substanzen, die einen starken Speichelfluss auslösen. Dafür sind insbesondere gut wasserlösliche, angenehm und süß schmeckende Hilfsstoffe geeignet, die aufgrund des osmotischen Effektes wirksam sind. Neben Saccharose, Glucose, Maltodextrin, sind insbesondere nicht kariogene wasserlösliche Hilfsstoffe wie Sorbit, Mannit, Xylit, Maltit, Malbit, Inulin und Isomalt geeignet. Die Wasserlöslichkeit bei 37°C beträgt mindestens 5% bevorzugt mehr als 15%. Aber auch Süßstoffe wie Aspartam, Acesulfam K, Nariumcyclamat, Saccharin und seine Salze, Sucralose und Neohesperidin erzeugen einen erstaunlichen spontanen Speichelfluss. Durch zugefügte Aromen wie z.B. Zitronenaroma, Grapefruitaroma wird z.T. reflektorisch spontan der Speichelfluss angeregt. Besonders interessant sind die Aromen unter den Namen Optaflow (Fa. Symrise), die z.B. nach zwei Minuten die Bildung einer höheren Speichelmenge auslösen als die allgemein bekannte Speichelbildung infolge saurer Zitronensäure. Eine lang anhaltende und verzögerte Speichelbildung ist insbesondere deshalb besonders vorteilhaft, da dadurch nach dem Schlucken der Hauptmenge der wirkstoffhaltigen Partikel weiterer Speichel gebildet wird, mit dem die zurückgebliebenen Partikel leicht geschluckt werden können.

Einen spontanen und starken Speichelfluss lösen auch wasserlösliche organische Säuren wie Weinsäure, Zitronensäure, Apfelsäure, Ascorbinsäure und dergleichen und deren wasserlösliche Salze, insbesondere deren Natrium- und Kaliumsalze wie beispielsweise Natrium- oder Kaliumhydrogentartrat, Natriumhydrogencitrat oder Natriumascorbat aus.

In zahlreichen Tests wurde festgestellt, dass die notwendige Speichelmenge zum Schlucken der Teilchen abhängig ist von der Menge an bereits im Mund vorhandenen Speichel und von der Teilchengröße der Partikel mit der erfindungsgemäßen weichen, aber stabilen Oberfläche. Leicht lassen sich Teilchen bis zu einer Größe von ca. 2,5 mm Durchmesser schlucken, wobei interessanterweise Teilchen etwa unterhalb 0,5 mm aufgrund ihrer Kleinheit eine höhere Adsorption an die Zunge und Mundschleimhaut aufweisen, so dass mehr Speichel zur vollständigen Entfernung der wirkstoffhaltigen Partikel aus der Mundhöhle notwendig ist. Auch bei Teilchen mit einem Durchmesser von ca. 2,5 mm und größer kommt es häufiger vor, dass einige dieser Teilchen nach dem ersten Schluckvorgang zurückbleiben, so dass auch hier mehr Speichel benötigt wird zu deren vollständigen Entfernung aus der Mundhöhle. Die Speichelmenge aus dem speichelfördernden Hilfsstoff ergibt sich aus dessen Zusammensetzung und dessen Menge. So ist es durchaus angebracht bei 50mg wirkstoffhaltigen Teilchen 1,5 bis 2,5 g eines gut schmeckenden speichelflussfördernden Hilfsstoffgemisches zuzufügen wie es auch möglich ist, mit dem von ca. 50 mg Zitronensäure ausgelösten Speichelfluss ca. 1 g wirkstoffhaltige Teilchen mit einer weichen, geglätteten Oberfläche weitgehendst quantitativ zu schlucken. Das Verhältnis der Masse der erfindungsgemäßen pharmazeutischen Zusammensetzung zur Masse der speichel bildenden Verbindung liegt damit bevorzugt bei 1:50 bis 10:1, bevorzugt 1:5 bis 1:1. Selbstverständlich können auch mehrere speichelbildende Verbindungen im Gemisch eingesetzt werden.

Enthält die erfindungsgemäße Beschichtung bei niedrigem pH-Wert lösliche Polymere, wie es z.B. bei Verwendung des erfindungsgemäß besonders bevorzugten Eudragit E als Bestandteil b)(ii) der Fall ist, kann es zu Schwierigkeiten kommen, wenn die speichelbildende Verbindung im Mund einen sehr niedrigen pH-Wert einstellt. Dann kann es vorteilhaft sein, in der erfindungsgemäßen Beschichtung noch einen basischen Bestandteil vorzusehen, wie z.B. Trinatriumcitrat oder Trinatiumphosphat und/oder die Menge an basischen Kohlendioxid-freisetzenden Verbindungen zu erhöhen. Dies verhindert die vorzeitige Auflösung der erfindungsgemäßen Beschichtung. Auch durch den pH-Wert des Speichels selbst wird innerhalb von Sekunden nach der Einnahme und dem gewünschten raschen Speichelfluss durch die Säure diese neutralisiert und der pH in einen Bereich angehoben, in dem das Eudragit E unlöslich ist.

Im Folgenden werden verschiedene bevorzugte Ausführungsformen der Erfindung beschrieben. Diese bevorzugten Ausführungsformen sind nicht einschränkend.

In einer bevorzugten Ausführungsform wird eine Suspension aus dem gelösten Eudragit E mit dem bevorzugten Polymer Polyacrylsäure (Carbopol 971 P) und bevorzugt alkalischen Verbindungen wie Natriumhydrogencarbonat, Kaliumhydrogencarbonat, Calciumcarbonat und Magnesiumcarbonat, die in Gegenwart von Säure rasch Kohlendioxid bilden, gemischt und auf die unebenen, oft scharfkantigen Kristalle oder Teilchen mit rauer Oberfläche aufgesprüht. Selbst wenn die Teilchen vereinzelt im Mund verbleiben, so besitzen sie noch nach 30 min eine stabile, weiche angenehm empfundene Oberfläche. Dieses Verhalten ist grundsätzlich anders als bei den Ausführungsformen des Standes der Technik, bei denen das gequollene Polymer bereits durch eine kurze Bewegung mit der Zunge von den Teilchen/Kernen abgerieben wird bzw. die Konzentration an quellendem Polymer auf der Partikeloberfläche so groß ist, dass die überzogenen Teilchen bei Kontakt mit Speichel zu einem hoch viskosen Pelletbrei zusammengeklebt werden oder an Gaumen und Zähnen kleben.

Das in das nicht-gelbildende Polymer eingearbeitete gelbildende Polymer ist bevorzugt sauer und reagiert bei Zutritt von Speichel mit den Bicarbonaten/Carbonaten unter Kohlendioxidbildung und Erhöhung des pH. Dabei steigt durch Salzbildung dieses Polymers seine Viskosität stark an und die entweichenden Kohlendioxidbläschen (ca. 50 µm Durchmesser) unterstützen die rasche Glättung durch Volumenzunahme der Teilchenoberfläche. Die Glättung bzw. Ausrundung der Teilchen mit ihrer scharfkantigen, im Mund als sandig, körnig empfundenen Oberfläche geschieht durch die infolge der Quellung der Polyacrylsäure und durch die austretenden CO₂-Mikrobläschen erzwungene Ausdehnung des Polymergerüstes, wobei gequollener Polymer aus dem Polymergerüst nicht austritt und keine gelartige oder klebrige Partikeloberfläche entsteht. Sobald eine klebrige und/oder gelartige Oberfläche sich bildet, ist die mechanische Stabilität einer weichen Partikeloberfläche ungenügend, d.h. wenige Bewegungen der Zunge über die Partikeloberfläche genügen, diese zu entfernen. Dieser mechanische Stress zwischen Partikeloberfläche und Zunge und zwischen Gaumen, Partikel und Zunge ist jedoch unvermeidlich, um praktisch alle Partikel in den Magen zu befördern. Gerade bei kleinen Kindern muss von einem unkontrollierbaren Stress auf die Partikeloberfläche ausgegangen werden. Bei den erfindungsgemäßen pharmazeutischen Zusammensetzungen weisen in der Mundhöhle zurückgebliebene Partikel auch nach 10 bis 20 Minuten noch eine weiche Partikeloberfläche auf und führen nicht durch eine körnige, sandige Empfindung zur Einnahme-Ablehnung.

Diese Eigenschaften der Teilchenoberfläche sind gegenüber dem Stand der Technik neu, da ein Zusammenkleben der Partikel vermieden wird bzw. eine Haftung der Partikel an Gaumen oder Zähnen praktisch nicht auftritt. Ganz besonders ist hervorzuheben, dass die weiche, in der Mundhöhle als angenehm empfundene Partikeloberfläche mechanisch stabil ist und auch durch Bewegung mit der Zunge nicht abgerieben wird. Im pH-Bereich des Speichels von ca. 5,0 bis 7,0 zeigt das gelbildende Polymer gemäß b)(i) bevorzugt seine höchste Viskosität. Mit sinkendem pH dagegen, insbesondere ab pH 3,5, löst sich bei der bevorzugten Ausführungsform, bei der das nicht-gelbildende Polymer b)(ii) im Magensaft löslich ist, immer rascher auf und die Viskosität des gelbildenden Polymers b)(i) geht ebenfalls zurück. Im sauren Magen unterstützen die im Überschuss vorhandenen CO₂-bildenden Hilfsstoffe den Auflöseprozess der erfindungsgemäßen Beschichtung, so dass die Wirkstofffreisetzung des Wirkstoffes praktisch nicht behindert wird. Dies ist ein besonderer Vorteil der erfindungsgemäßen Beschichtung, da sie trotz stabilen, angenehmen Mundgefühls der Teilchen und sehr guter Speichelresistenz bei Absenken des pH-Wertes den Wirkstoff ungehindert freisetzen kann (sofern nicht durch eine unter der erfindungsgemäßen Schicht aufgebrachte Schicht eine Steuerung der Wirkstofffreigabe aus dem Kern erfolgen soll).

Die Schnelligkeit der Bildung einer weichen, aber stabilen Teilchenoberfläche wird in erfinderischer Weise durch das Quellungsvermögen, die Quellgeschwindigkeit der verwendeten Polymere, durch die Menge an zugesetztem Carbonat/Hydrogencarbonat, das Verhältnis nicht-gelbildendes Polymer/gelbildendes Polymer und die Menge zur Verfügung stehender Säure zur CO₂-Bildung gesteuert. Ist das eingesetzte gelbildende Polymer nicht ausreichend sauer oder will man die Kohlendioxidbildung beschleunigen, kann die Ausbildung einer weichen Partikeloberfläche durch den Zusatz einer geringen Menge Säure unterstützt werden, die mit dem im Überschuss vorhandenen Hydrogencarbonat/Carbonat eine weitere Kohlendioxidbildung verursacht. Die winzigen Gasbläschen blasen sozusagen die Schicht aus nicht-gelbildendem Polymer und gelbildendem Polymer auf und unterstützen die Ausbildung einer weichen, aber mechanisch stabilen im Mund als angenehm empfundenen Teilchenoberfläche. Da der Speichel selbst in der Regel neutral bis schwach alkalisch ist und außerdem Hydrogencarbonate/Carbonate vorhanden sind, besteht im Falle des Einsatzes von säurelöslichen Eudragit E nicht die Gefahr, dass dieses Polymer bereits im Mund aufgelöst wird. Die geringen Mengen an Säure reagieren bevorzugt mit den im Überschuss vorhandenen Hydrogencarbonaten/Carbonaten und werden dabei in Bruchteilen von Sekunden unter CO₂-Bildung neutralisiert.

Bei Einsatz eines Polymers als Bestandteil b)(ii), das im Magensaft löslich ist, wie Eudragit E, wird zur Vermeidung eines vorzeitigen Auflösens im Mund das Verhältnis aus Polymer b)(ii), Polymer b)(i), CO₂-freisetzender Verbindung und optional saurer Verbindung bevorzugt so eingestellt, dass eine 3%ige wässrige Dispersion aus den genannten Komponenten mindestens einen pH-Wert von 5,5 aufweist. Bei speichelresistenten, im Sauren nicht löslichen Polymeren als Bestandteil b)(ii) dagegen ist der pH-Wert einer 3%igen wässrigen Dispersion der genannten Komponenten so eingestellt, dass ein pH von bevorzugt mindestens 4 erreicht wird. Der Grund für diese Einstellung liegt im Wesentlichen darin, dass die genannten gelbildenden Polymere unter einem pH-Wert von 4 in wässriger Lösung einen deutlichen Viskositätsabfall aufweisen.

In der Regel werden die genannten nicht-gelbildenden Polymere b)(ii) in einem organischen Lösungsmittel wie Ethanol, Isopropanol, Aceton oder Mischungen gelöst. Die gelbildenden Polymere b)(i), die CO₂-freisetzend Verbindung, gegebenenfalls die genannten sauren, bevorzugt mikronisierten Verbindungen und weitere typische Hilfsstoffe, die bei der Herstellung von Beschichtungen eingesetzt werden wie z.B. Weichmacher und Antiklebemittel wie Talkum, Farbstoffe wie Eisenoxidpigmente, Titandioxid werden in den Lösungen des nicht-gelbildenden Polymers b)(ii) suspendiert. Die Suspensionen werden in bekannter Weise bevorzugt in der Wirbelschicht auf die fluidisierten Wirkstoffkerne aufgesprüht. Der Coatingprozess verläuft unproblematisch unter bekannten Bedingungen für Zuluftmenge, Zulufttemperatur, Sprühgeschwindigkeit.

Es ist jedoch auch möglich, die zu überziehenden Wirkstoffkerne in einem geeigneten Gerät, z.B. einem Spheronizer in starke Bewegung zu setzen und mit der Lösung des nicht-gelbildenden Polymers b)(ii) zu besprühen. Sobald alle Teilchen gleichmäßig mit der Lösung befeuchtet sind, wird in sehr kleinen Portionen die mikronisierte Pulvermischung aus dem gelbildenden Polymer b)(i), den CO₂-freisetzenden Verbindungen, Antiklebemitteln, Farbpigmenten und gegebenenfalls mikronisierter Säure auf die Partikel aufgeklebt und mit weiterer Lösung besprüht. Dieser Vorgang wird solange wiederholt, bis die gesamte Pulvermenge aufgetragen ist. Auch auf diese Weise ist es möglich, das gelbildende Polymer b)(i) und die Hilfsstoffe in das Gerüst aus dem nicht-gelbildende Polymer b)(ii) ausreichend homogen einzuarbeiten, so dass nach Trocknung und Zutritt von Speichel ebenfalls sehr rasch eine weiche, mechanisch stabile Partikeloberfläche im Mund entsteht.

Erfindungsgemäß kann der Fachmann das Ausmaß der Partikel-Volumenzunahme im Mund, die empfundene Weichheit der Partikeloberfläche und die mechanische Stabilität der weichen Partikeloberflächen in verschiedenster Weise steuern. Ist zum Beispiel eine sehr rasche Bildung einer weichen Oberfläche gewünscht, wird er das Verhältnis zwischen nicht-gelbildendem Polymer b)(ii) und gelbildendem Polymer b)(i) zugunsten des gelbildenden Polymers erhöhen und hierzu ein rasch und intensiv gelbildendes Polymer einsetzen. Alternativ oder zusätzlich kann der Fachmann auch die Menge an CO₂-bildenden Verbindungen und die Menge an Säure in der Beschichtung erhöhen. Sollen dagegen bei besonders bitteren Wirkstoffen z.B. auch nach einer Stunde im Mund ganz vereinzelt auftretende Partikel noch eine weiche, angenehm empfundene und den Schluckvorgang fördernde Oberfläche aufweisen, so wird er z.B. die Menge an nicht-gelbildendem Polymer b)(ii) zugunsten des gelbildenden Polymers b)(i) vergrößern, kein saures Polymer einsetzen oder die Säuremenge zur Bildung von CO₂ reduzieren. Er kann jedoch auch ein gelbildendes Polymer b)(i) einsetzen, für das eine langsame Gelbildungsgeschwindigkeit charakteristisch ist. Um jedoch nach der Teilcheneinnahme eine sich rasch ausbildende, aber auch lang anhaltende weiche Teilchenoberfläche zu erhalten, könnte der Fachmann als gelbildendes Polymer b)(i) eine Kombination von langsam und schnell gelbildenden Polymeren einsetzen. In einem solchem Fall ist z.B. für das Polymer b)(i) die Kombination aus dem schnell und intensiv gelbildenden Carbopol 971 P mit einem langsam gelbildenden aber hochviskosen Celluloseether, Methylhydroxypropylcellulose KM 100 (Methocel KM 100) geeignet. In jedem Fall ist es nicht erwünscht, dass eine klebrige bis gelartige und stark gequollene, schwach haftende Schicht auf den Teilchen entsteht, weil diese durch die Bewegung der Zunge und nach kurzer Verweildauer in der Mundhöhle sich von den Teilchen vollständig ablösen kann und die unangenehm empfundene, körnige, sandartige Teilchenoberfläche wieder zum Vorschein kommt.

Erfindungsgemäß ist es ebenfalls möglich, z.B. in die erfindungsgemäße Beschichtung mikronisiertes Kaliumhydrogencarbonat und mikronisiertes Calciumcarbonat einzusetzen. Bei Einnahme wird aufgrund der sehr hohen Wasserlöslichkeit das Kaliumhydrogencarbonat sofort z.B. mit dem sauren Polymer reagieren und zur gewünschten Glättung beitragen, wohingegen Calciumcarbonat bevorzugt mit der wesentlich azideren Magensäure nach dem Schlucken der Teilchen reagieren wird und so für eine rasche Wirkstofffreisetzung durch schnelle Zerstörung der weichen erfindungsgemäßen Beschichtung infolge CO₂-Bildung beitragen wird.

Erfindungsgemäß ist es möglich, dass der Fachmann die Dicke, die Geschwindigkeit der Ausdehnung, die Weichheit, die mechanische Stabilität der erfinderischen, angenehm im Mund empfundenen Beschichtung beeinflussen kann und dass die Zusammensetzung der gewählten Beschichtung zusätzlich einen wesentlichen Beitrag zu ihrer Zerstörung unter dem Einfluss von Magensaft und damit zu einer raschen Wirkstofffreisetzung leisten kann.

Es können erfindungsgemäß auch Mischungen von rasch freisetzenden und z.B. retardierten Wirkstoffteilchen mit der erfindungsgemäßen Beschichtung versehen werden. In der Regel jedoch handelt es sich um wirkstoffhaltige Teilchen, die nach der direkten Applikation in den Mund den Wirkstoff möglichst rasch im Magen freisetzen sollen. Deshalb ist es sehr wichtig, dass die erfindungsgemäße Beschichtung trotz eines optimalen Mundgefühls die Wirkstofffreisetzung im Magen möglichst nicht behindert, sondern sogar fördert. Dies ist bei den bekannten speichelresistenten Beschichtungen, die geringe Mengen eines wasserlöslichen Polymers enthalten, nicht gesichert. Das wasserlösliche Polymer hat bei diesen bekannten Ausführungsformen allein die Funktion, die Wirkstofffreisetzung zu beeinflussen. Im Stand der Technik werden hierzu in der Regel praktisch nicht-gelbildende, aber ausschließlich wasserlösliche Polymere wie Hydroxypropylmethylcellulosen eingesetzt, die als 2%ige Lösungen eine Viskosität von maximal 50, besser 3-6 mPa·s, aufweisen. In keinem Fall wird die rasche Glättung der Partikeloberfläche unter Ausbildung einer weichen, im Mund als angenehm empfundenen, mechanisch stabilen Beschichtung erreicht oder bezweckt.

Im Stand der Technik wird häufig als speichelresistentes Polymer Ethylcellulose eingesetzt. Dieses ist im Magen unlöslich, so dass selbst durch den Einbau eines wasserlöslichen Polymers in die Ethylcellulose die rasche Wirkstofffreisetzung verzögert ist, wenn nicht sehr viel wasserlösliches Polymer verwendet wird, wodurch jedoch eine wirksame Speichelresistenz der Schicht aufgehoben würde. Erfindungsgemäß sorgt auch die CO₂-bildendende Verbindung, die nicht nur im Mund sondern auch im Magen reagiert, dafür, dass die erfindungsgemäße Beschichtung im Magen entweder zerstört wird oder soweit aufquillt, dass sie die Freisetzung des Wirkstoffs nicht beeinflusst. Befindet sich unter der erfindungsgemäßen Beschichtung z.B. eine weitere, die Wirkstofffreigabe steuernde Retardschicht, so wird infolge der raschen Ablösung der erfindungsgemäßen Beschichtung im Magen die gewünschte Wirkstoffreissetzung entsprechend der Retardschicht praktisch nicht beeinflusst. Dies gilt in gleicher Weise z.B. auch für Teilchen, die unter der erfindungsgemäßen Beschichtung eine magensaftresistente Schicht aufweisen.

Das Verhältnis zwischen den Komponenten nicht-gelbildendes Polymer b)(ii), gelbildendes Polymer b)(i), CO₂-bildender Verbindung und einer optional noch einsetzbaren Säure wird geeigneterweise so gewählt, dass im Mund des Patienten das gelbildendes Polymer b)(i) mit Unterstützung der CO₂-Mikrobläschen das Polymergerüst des nicht-gelbildenden Polymers b)(ii) aufweitet, aber in dem Polymergerüst eingeschlossen verbleibt. Das gelbildende Polymer tritt nicht aus der speichelresistenten, gequollenen Beschichtung aus und erzeugt an der Oberfläche keinen klebenden Effekt, der die Teilchen im Mund zusammenklebt. Es war jedoch für den Fachmann überraschend, dass trotzdem innerhalb von 30 Sekunden eine Volumenzunahme der Teilchen um 30% erfolgen kann und eine weiche und schluckfreundliche, im Mund als angenehm empfundene geglättete Oberfläche hervorgerufen wird, die dem mechanischen Stress innerhalb des Mundes über mehr als 30 Minuten widerstehen kann. Die Volumenzunahme beträgt in Wasser (pH 7,0, 37°C) bevorzugt mindestens 50%, stärker bevorzugt etwa 100% nach 4 Minuten.

Die erfindungsgemäße pharmazeutische Zusammensetzung kann beispielsweise mit etwas Wasser direkt in den Mund eingebracht werden. Bevorzugt wird die erfindungsgemäße pharmazeutische Zusammensetzung aber mit der speichelbildenden Komponente gemischt und bevorzugt in sogenannte Stick Packs abgefüllt und dann aus den Stick Packs ohne Wasser direkt in den Mund, bevorzugt auf die Zungenmitte, plaziert. Um eine hohe Dosisgenauigkeit zu erhalten, können auch ein oder mehrere Wirkstoff/Wirkstoffe enthaltende erfindungsgemäße Teilchen von der speichelbildenden Mischung getrennt in die Stick Packs abgefüllt werden. Spezielle Dosiereinrichtungen können gleichzeitig wirkstoffhaltige Teilchen separat von der speichelbildenden Mischung in den gleichen Stick Pack abfüllen. Die erfindungsgemäß überzogenen wirkstoffhaltigen Teilchen und die speichelbildende Mischung können aber auch gemischt oder separat in spezielle Hartgelatine-Kapseln oder z.B. in Satchets abgefüllt werden, die leicht zu öffnen sind. Der Inhalt wird dann von dem Patienten direkt auf die Zunge geschüttet. Grundsätzlich sind aber so genannte Stick Packs als Primärpackmittel besonders bevorzugt, da die aus Aluminium kaschierter Folie hergestellten Stick Packs vollkommen dicht sind und den Inhalt vor Feuchtigkeit perfekt schützen. Außerdem kann aus den länglichen und ca. nur 1 bis 2 cm breiten Stick Packs nach Öffnen der Inhalt sehr gut direkt in den Mund platziert werden. Das Medikament kann aber auch aus einer Mischung von erfindungsgemäß hergestelltem Wirkstoff enthaltenden Teilchen und der speichelbildenden Komponente bestehen, die z.B. in Dosen oder Flaschen abgefüllt wird. Mit Hilfe eines Dosierlöffels wird dann die Mischung in den Mund geschüttet. Erfindungsgemäß ist es ebenfalls möglich, dass die erfindungsgemäße pharmazeutische Zusammensetzung und die speichelbildende Verbindung getrennt verpackt werden, mit der Anweisung, dass sie gemeinsam verabreicht werden sollen.

Schließlich können die erfindungsgemäßen Teilchen aber auch in ein Glas Wasser geschüttet werden, wobei sie sich problemlos verteilen und durch Volumenzunahme Auftrieb bekommen, wodurch sich die schwebenden Teilchen mit der Flüssigkeit leicht trinken lassen.

Die Erfindung wird durch nachfolgende Beispiele weiter veranschaulicht.

### Beispiel 1

1a) Herstellung von Guaifenesin-Pellets

| | | |
|---|---|---|
| Guaifenesin | (1) | 100,0 kg |
| Mikrokristalline Cellulose | (2) | 102,0 kg |
| Povidone K25 | (3) | 8,0 kg |

Die drei Komponenten (1) bis (3) werden in einem geeigneten Feuchtmischgerät gemischt und mit 30 kg Wasser befeuchtet. Die entstandene plastische Masse wird extrudiert und in einem Spheronizer zu Pellets in einem Korngrößenbereich von 0,4 bis 1,6 mm gerundet.
1b) Herstellung von speichelresistenten Guaifenesin-Pellets
Nach Trocknung werden die Teilchen mit nachfolgender Coatingschicht überzogen:

| | | |
|---|---|---|
| Eudragit E | (1) | 12,1 kg |
| Triethylcitrat | (2) | 1,0 kg |
| Talk | (3) | 22,1 kg |
| Magnesiumstearat | (4) | 1,4 kg |
| Ethanol | (5) | 101,0 kg |
| Wasser | (6) | 8,0 kg |

In einer Mischung aus (5) und (6) werden (1) und (2) gelöst und (3) und (4) suspendiert. Die Suspension wird in bekannter Weise auf die 210 kg Guaifenesin-enthaltenden Teilchen aus Beispiel 1a) aufgesprüht (= 246,6 kg Guaifenesin-Pellets, speichelresistent).
1c) Herstellung von Guaifenesin-Pellets mit erfindungsgemäßer Beschichtung

| | | |
|---|---|---|
| Eudragit E | (1) | 9,0 kg |
| Carbopol 971P | (2) | 7,2 kg |
| Talk | (3) | 3,7 kg |
| Stearinsäure | (4) | 1,8 kg |
| Natriumhydrogencarbonat | (5) | 7,2 kg |
| Ethanol | (6) | 116,0 kg |

In Ethanol (6) werden (1) und (4) gelöst und (2), (3) und (5) suspendiert. Die Suspension wird auf die 246,6 kg speichelresistente Guaifenesin-Pellets von Beispiel 1b) in bekannter Weise in einer Wirbelschichtapparatur aufgesprüht. Nach der Trocknung erhält man 275,5 kg Guaifenesin-Pellets gemäß der Erfindung (10,5% erfindungsgemäße Beschichtung).
1d) Die in 1c) hergestellten erfindungsgemäßen Teilchen weisen eine weiche, im Mund als angenehm empfundene aber mechanisch stabile Schicht auf, die selbst nach 30
Minuten noch voll erhalten ist und auch einem mechanischen Stress durch Reiben mit der Zunge widersteht. Die für den Fachmann überraschend schnell erfolgende Glättung der Teilchenoberfläche wird in dem folgenden Versuch demonstriert.

In einen 250-ml-Messzylinder, der mit Wasser von ca. 37°C gefüllt ist, wird eine gewisse Menge der in Beispiel 1c) hergestellten Teilchen geschüttet und innerhalb 5 Sekunden das Volumen der abgesetzten Teilchen bestimmt. Nach gewissen Zeitabständen wird die infolge der Ausdehnung und Glättung der Partikeloberfläche eintretende Volumenzunahme bestimmt.

| Partikelvolumen in Wasser, 37°C | | | | | | |
|---|---|---|---|---|---|---|
| Start | 0,5 min | 1 min | 2 min | 3 min | 4 min | 5 min |
| 70 ml | 93 ml | 100 ml | 116 ml | 128 ml | 136 ml | 142 ml |
| 72 ml | 98 ml | 104 ml | 118 ml | 132 ml | 140 ml | 144 ml |
| 68 ml | 96 ml | 106 ml | 120 ml | 134 ml | 142 ml | 142 ml |
| 75 ml | 88 ml | 108 ml | 116 ml | 138 ml | 146 ml | 156 ml |
| 70 ml | 92 ml | 104 ml | 112 ml | 140 ml | 145 ml | 148 ml |
| Mittelwerte | | | | | | |
| 71 ml | 93 ml | 104 ml | 116 ml | 134 ml | 142 ml | 147 ml |

Wird der Versuch mit den nicht erfindungsgemäß beschichteten Teilchen aus Beispiel 1b) wiederholt, steigt das Teilchenvolumen von zunächst 70 ml nach 4 Minuten nur auf 72 ml an.

Es ist für den Fachmann unerwartet, dass bereits im Mittel nach 30 Sekunden eine Volumenzunahme infolge Ausdehnung und Glättung der Teilchenoberflächen von über 30% eintritt und bereits nach 3 bis 4 Minuten sich das Volumen der Teilchen um 100% vergrößert hat. Betrachtet man sich die Volumenzunahme der Teilchen unter dem Mikroskop, so sind glatte, ebene Teilchenoberflächen zu erkennen, aus denen vereinzelt Gasbläschen in der Größe von ca. 50 µm austreten. Im Mund selbst werden die Partikel nicht als Fremdkörper empfunden, da die Oberflächen glatt, weich und duktil sind aber mechanisch so stabil, dass die geglättete Oberfläche durch Bewegung mit der Zunge oder durch Andrücken an den Gaumen nicht entfernt werden kann. Das angenehme Mundgefühl besitzen selbst noch Partikel, die vereinzelt zufälligerweise zwischen die Zähne geraten sind und nach 30 Minuten wieder im Mundraum auftreten.

Die erfindungsgemäßen Teilchen dehnen sich damit in Wasser von 37°C innerhalb von 30 Sekunden unter einer Volumenzunahme von ca. 30% aus und bilden eine weiche, glatte Pelletoberfläche. Die Pellets sind einzeln dispergiert und kleben nicht zusammen. Sie haben eine Tendenz zum Aufschwimmen. Entnimmt man die Teilchen aus dem Wasser nach 5 Minuten, so lässt sich die erfindungsgemäße Beschichtung durch leichten Druck mit einem Spatel nicht zerstören. Erst bei einem deutlichen mechanischen Stress durch Reiben zwischen Daumen und Zeigefinger kann man die glatte, weiche äußere Schicht von dem harten inneren Wirkstoffkern wegreiben.

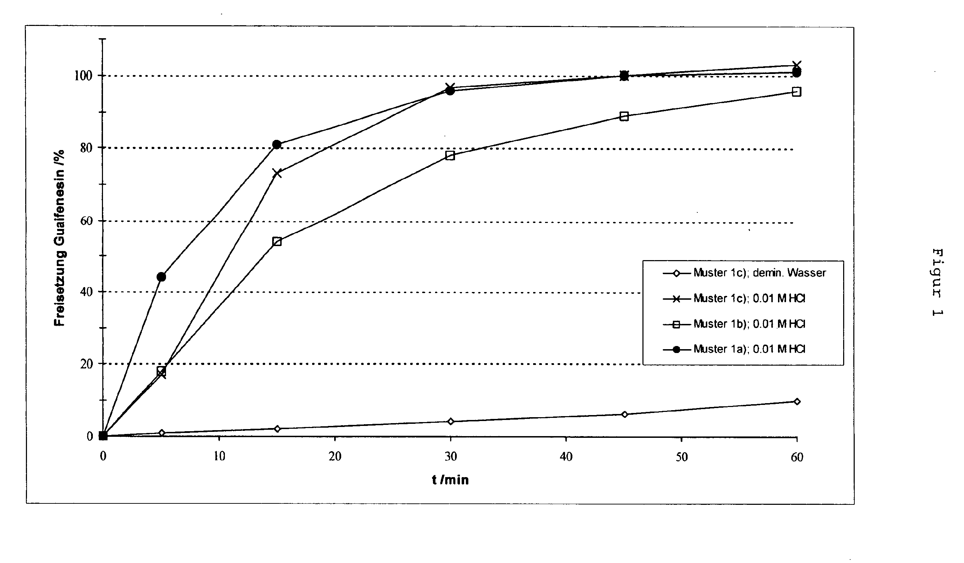

Nimmt man die Teilchen in den Mund, so verspürt man spätestens nach 30 Sekunden mit der Zunge eine ebene, weiche, glatte, Oberfläche ohne jedes sandige, körnige Gefühl, dass man sofort verspürt, wenn man die speichelresistenten Guaifenesin-Pellets 1b) ohne erfindungsgemäße Beschichtung in den Mund nimmt. Selbst nach 30 Minuten haben die erfindungsgemäßen Teilchen 1c) noch eine sehr angenehm empfundene, aber gegenüber der Berührung mit der Zunge stabile, weiche, unverletzte Oberfläche.
1e) Gemäß der US Pharmacopoe, Paddle-Methode, 50 Umdrehungen/Minute wurde in künstlichem Magensaft ein Dissolution-Test durchgeführt. Das Ergebnis ist in Figur 1 gezeigt, in der die Freisetzungen von Guaifenesin Pellets 1a), nicht erfindungsgemäß beschichteten Pellets 1b) und der erfindungsgemäßen Pellets 1c) dargestellt sind.

Die Wirkstofffreisetzung der erfindungsgemäßen Guaifenesin-Teilchen 1c) ist überraschenderweise gegenüber den speichelresistenten Teilchen 1b) trotz der zusätzlichen Beschichtung nicht reduziert. Aufgrund der Volumenzunahme der Teilchen 1c) beginnen diese aufzuschwimmen, so dass sie durch das drehende Paddle intensiver im Dissolution-Medium bewegt werden und dadurch überraschend gegenüber 1b) sogar eine raschere Wirkstofffreisetzung zeigen.

Die Guaifenesin-Freisetzung in Wasser ist wie gewünscht sehr gering. Selbst bei einer Verweilzeit von 5 Minuten in Wasser wird gerade ca. 1% Wirkstoff freigesetzt.

### Beispiel 2

Erfindungsgemäße Speichelfluss-fördernde Komponenten

| | | |
|---|---|---|
| Sorbitol, 0,1-1,0 mm | (1) | 558,0 kg |
| Natriumcarboxymethylcellulose | (2) | 10,0 kg |
| Aspartam | (3) | 2,0 kg |
| Tutti-Frutti Aroma (650916, Fa. Symrise) | (4) | 7,0 kg |
| Bananen-Aroma (214746, Fa. Symrise) | (5) | 2,0 kg |
| Magnesiumstearat | (6) | 3,0 kg |

werden durch ein Sieb der Maschenweite 1,5 mm gesiebt und 15 Minuten gemischt.

Die bei Einnahme von 582 mg speichelflussfördernder Masse gemäß Beispiel 2 gebildete Speichelmenge wurde an 10 Probanden geprüft. Sie betrug 30 Sekunden nach Einnahme 2,3 ml (1,8 bis 3,2 ml Spannweite). Sie ist zum Schlucken von 278 mg erfindungsgemäßen Guaifenesin-Teilchen 1 c völlig ausreichend.

### Beispiel 3

Stick Pack-Abfüllung
275,5 kg der Guaifenesin-Pellets 1c) werden mit 1 kg Magnesiumstearat und 1,5 kg Talkum gemischt. Auf einer Abfüllmaschine werden mittels eines 2-Kammer Dosierschiebers in einem Arbeitsgang 278 mg dieser Mischung mit 582 mg der Speichelfluss-fördernden Komponente gemäß Beispiel 2 in ein Stick Pack gefüllt und verschlossen. Das Füllgewicht beträgt 860 mg und enthält 100 mg Guaifenesin. Das Verhältnis der erfindungsgemäßen Guaifenesin-Pellets zur Speichelfluss-fördernden Mischung beträgt 1:2,1.

### Beispiel 4

Exakt nach Beispiel 1a) und 1b) werden 171 kg speichelresistente Guaifenesin-Pellets hergestellt, die 50 kg Guaifenesin enthalten.

### Beispiel 5

Herstellung von Guaifenesin-Pellets mit erfindungsgemäßer Beschichtung:

| | | |
|---|---|---|
| Guaifenesin-Pellets (nach Beispiel 4) | (1) | 171 kg |
| Eudragit E | (2) | 5,9 kg |
| Carbopol 971P | (3) | 4,7 kg |
| Talkum | (4) | 2,5 kg |
| Stearinsäure | (5) | 1,2 kg |
| Natriumbicarbonat | (6) | 4,7 kg |
| Ethanol | (7) | 75,0 kg |

Exakt nach Beispiel 1c) wird die Dispersion aus den Bestandteilen hergestellt und die speichelresistenten Guaifenesin-Pellets gemäß Beispiel 4 besprüht = 190 kg erfindungsgemäße Pellets (10,6% erfindungsgemäße Beschichtung). Sie werden mit 1 kg Magnesiumstearat und 1 kg Talkum gemischt.

### Beispiel 6

Erfindungsgemäße Speichelfluss-fördernde Mischung

| | | |
|---|---|---|
| Sorbitol, 0,1 bis 1,0 mm | (1) | 276,0 kg |
| Natriumcarboxymethylcellulose | (2) | 5,0 kg |
| Weintrauben-Aroma (208275, Fa. Symrise) | (3) | 3,0 kg |
| Himbeer-Aroma (652742, Fa. Symrise) | (4) | 1,0 kg |
| Aspartam | (5) | 1,0 kg |
| Magnesiumstearat | (6) | 3,0 kg |

Entsprechend Beispiel 2 und 3 werden pro Stick Pack 192 mg der erfindungsgemäßen Pellets nach Beispiel 5 mit 289 mg der Speichelfluss-fördernden Mischung nach Beispiel 6 in ein Stick Pack gefüllt. Das Füllgewicht beträgt 481 mg und enthält 50 mg Guaifenesin. Das Verhältnis der erfindungsgemäßen Guaifenesin-Pellets zur Speichelfluss-fördernden Mischung beträgt 1:1,5.

Innerhalb von ca. 30 Sekunden nach der oralen Einnahme bildet sich im Mund ca. 1,5 ml Speichel, der vollkommen ausreichend ist zum Schlucken von 192 mg Guaifenesin-Pellets gemäß der Erfindung.

### Beispiel 7

7a) Koffein-Teilchen mit speichelresistenter Schicht
Kompaktierte Koffein-Teilchen mit einer mittleren Teilchengröße von 0,3 mm werden mit einer Eudragit-Beschichtung exakt nach Beispiel 1b) überzogen. Die Masse der Beschichtung beträgt 10,8%.
7b) Herstellung von Koffein-Teilchen mit erfindungsgemäßer Beschichtung

| | | |
|---|---|---|
| Koffein-Partikel 7a) | (1) | 4000 g |
| Eudragit E | (2) | 300 g |
| Carbopol 971 P | (3) | 240 g |
| Talkum | (4) | 125 g |
| Stearinsäure | (5) | 60 g |
| Natriumhydrogencarbonat | (6) | 240 g |
| Ethanol | (7) | 3800 g |

Eine Suspension wird entsprechend Beispiel 1c) hergestellt und in bekannter Weise in einer Wirbelschicht-Apparatur aufgesprüht. Nach der Trocknung erhält man 4965 g Koffein-Partikel gemäß der Erfindung (19,4% erfindungsgemäße Beschichtung).
Das Volumen der Teilchen in Wasser von 37°C dehnt sich innerhalb von 30 Sekunden um 35% aus. Bereits nach drei Minuten ist die Endausdehnung mit 85% erreicht. Nimmt man die Teilchen in den Mund, spürt man innerhalb von 15 Sekunden bei Berührung mit der Zunge oder Drücken gegen den Gaumen eine angenehme weiche, aber mechanisch stabile, glatte Oberfläche. Die Teilchen werden nicht als Fremdpartikel im Mund empfunden und lassen sich besonders leicht schlucken. Vereinzelte im Mund zurückgebliebene Teilchen schmecken nicht bitter und zeigen bei Berührung mit der Zunge oder dem Gaumen eine weiche, unverletzte Oberfläche.
Im Dissolution-Test, beschrieben in Beispiel 1e), erhält man mehr als 90% Freisetzung von Koffein nach 15 Minuten in künstlichem Magensaft. Die Teilchen zeigen dabei eine deutliche Tendenz zum Aufschwimmen aufgrund der Volumenzunahme der Teilchen.
7c) Erfindungsgemäße Speichelfluss-fördernde Komponente

| | | |
|---|---|---|
| Sorbitol 0,1 bis 1,0 mm | (1) | 14,0 kg |
| Natriumcarboxymethylcellulose | (2) | 0,3 kg |
| Sucralose | (3) | 0,06 kg |
| Cappuccino-Aroma (142618, Fa. Symrise) | (4) | 0,1 kg |
| Magnesiumstearat | (5) | 0,15 kg |
| Talkum | (6) | 0,15 kg |

werden durch ein Sieb der Maschenweite 1,5 mm gesiebt und 15 Minuten gemischt.
7d) Stick Pack-Abfüllung
4965 g der erfindungsgemäßen Koffein-Teilchen 7b) werden mit 50 g Magnesiumstearat und 50 g Talkum gemischt. Auf einer Abfüllmaschine werden mittels eines Zweikammer-Dosierschiebers in einem Arbeitsgang 284 mg dieser Mischung mit 828 mg der Speichelfluss-fördernden Komponente 7d) in ein Stick Pack gefüllt und verschlossen. Das Gesamtfüllgewicht beträgt 1112 mg und enthält 200 mg Koffein. Das Verhältnis der erfindungsgemäßen Koffein-Teilchen zur Speichelfluss-fördernden Mischung beträgt 1:2,9.

### Beispiel 8

8a) Herstellung von Guaifenesin/Dextromethorphan-Pellets

| | | |
|---|---|---|
| Guaifenesin | (1) | 100,0 kg |
| Dextromethorphan | (2) | 5,0 kg |
| Mikrokristalline Cellulose | (3) | 100,0 kg |
| Polyethylenglykol 6000 | (4) | 10,0 kg |
| Povidone K 25 | (5) | 8,0 kg |

Die vier Komponenten (1 bis 4) werden in einem geeigneten Feuchtmischgerät gemischt und mit 23 kg Wasser befeuchtet. Die entstandene Masse wird extrudiert und in einem Spheronizer zu Pellets in einem Korngrößenbereich von 0,6 bis 1,6 mm gerundet.
8b) Herstellung von speichelresistenten Guaifenesin/Dextromethorphan-Pellets
Nach dem Trocknen werden 223 kg 8a) mit nachfolgender Beschichtung überzogen:

| | | |
|---|---|---|
| Eudragit E | (1) | 10,4 kg |
| Triethylcitrat | (2) | 0,5 kg |
| Talkum | (3) | 6,0 kg |
| Magnesiumstearat | (4) | 1,2 kg |
| Ethanol | (5) | 75,0 kg |
| Wasser | (6) | 5,0 kg |

Entsprechend Beispiel 1b) wird die Suspension in bekannter Weise auf 223 kg Guaifenesin/Dextromethorphan-Teilchen aufgesprüht = 241,1 kg Guaifenesin/Dextromehorphan-Pellets, speichelresistent.
8c) Herstellung der Guaifenesin/Dextromethorphan-Pellets mit erfindungsgemäßer Beschichtung

| | | |
|---|---|---|
| Eudragit E | (1) | 10,0 kg |
| Carbopol 971 P | (2) | 11,0 kg |
| Talkum | (3) | 3,0 kg |
| Stearinsäure | (4) | 1,5 kg |
| Natriumhydrogencarbonat | (5) | 10,0 kg |
| Ethanol | (6) | 106,0 kg |

Entsprechend dem Beispiel 1d) wird die hergestellte Suspension auf 241,1 kg speichelresistente Guaifenesin/Dextromethorphan-Pellets in bekannter Weise in einer Wirbelschicht-Apparatur aufgesprüht. Nach Trocknung erhält man 276,6 kg Pellets gemäß der Erfindung (12,8% erfindungsgemäße Beschichtung). Die erfindungsgemäßen Pellets dehnen sich in Wasser von 37°C innerhalb von 30 Sekunden unter einer Volumenzunahme von 39% aus und bilden eine weiche, glatte Pelletoberfläche. Die Pellets sind einzeln dispergiert und kleben nicht zusammen. Im Mund spürt man bereits nach 5 Sekunden eine weiche, glatte, mechanisch stabile Oberfläche, die durch Berührung mit der Zunge oder Drücken gegen den Gaumen nicht zerstört werden kann. Die Partikel werden im Mund nicht als Fremdkörper empfunden und lassen sich leicht schlucken.
8d) Erfindungsgemäße Speichelfluss-fördernde Komponente

| | | |
|---|---|---|
| Sorbitol, 0,1 bis 1,0 mm | (1) | 558,0 kg |
| Natriumcarboxymethylcellulose | (2) | 10,0 kg |
| Aspartam | (3) | 2,0 kg |
| Tutti-Frutti Aroma (650916, Fa. Symrise) | (4) | 7,0 kg |
| Bananen-Aroma (214746, Fa. Symrise) | (5) | 2,0 kg |
| Magnesiumstearat | (6) | 3,0 kg |

werden durch ein Sieb der Maschenweite 1,5 mm gesiebt und 15 Minuten gemischt.
8e) Stick Pack-Abfüllung
276,6 kg der Guaifenesin/Dextromethorphan-Pellets 8c) werden mit 1 kg Magnesiumstearat und 1,4 kg Talkum gemischt. Auf einer Abfüllmaschine werden mittels eines 2-Kammer Dosierschiebers in einem Arbeitsgang 279 mg dieser Mischung mit 582 mg der Speichelfluss-fördernden Komponente gemäß Beispiel 3 in ein Stick Pack gefüllt und verschlossen. Das Füllgewicht beträgt 861 mg und enthält 100 mg Guaifenesin und 5 mg Dextromethorphan. Das Verhältnis der erfindungsgemäßen Guaifenesin/Dextromethorphan-Pellets zur Speichelfluss-fördernden Mischung beträgt 1:2,1.
In gleicher Weise füllt man in einem Arbeitsgang 139,5 mg Pellets mit 291 mg der Speichelfluss-fördernden Komponente ab. Das Gesamtfüllgewicht beträgt 430,5 mg und enthält 50 mg Guaifenesin und 2,5 mg Dextromethorphan.
8f) Im Dissolution-Test gemäß der Pharmakopoe, Paddle-Methode, 50 Umdrehungen/Minute im künstlichen Magensaft wird sowohl Guaifenesin als auch Dextromethorphan innerhalb von 20 Minuten zu mehr als 90% freigesetzt. Die Pellets zeigen eine deutliche Tendenz zum Aufschwimmen.

### Beispiel 9

9a) Herstellung von Guaifenesin/Phenylephrin-Pellets

| | | |
|---|---|---|
| Guaifenesin | (1) | 100,0 kg |
| Phenylephrin | (2) | 5,0 kg |
| Mikrokristalline Cellulose | (3) | 100,0 kg |
| Polyethylenglykol 6000 | (4) | 10,0 kg |
| Povidone K 25 | (5) | 8,0 kg |

Die vier Komponenten (1 bis 4) werden in einem geeigneten Feuchtmischgerät gemischt und mit 23 kg Wasser befeuchtet. Die entstandene plastische Masse wird extrudiert und in einem Spheronizer zu Pellets in einem Korngrößenbereich von 0,6 bis 1,6 mm gerundet.
9b) Herstellung von speichelresistenten Guaifenesin/Phenylephrin-Pellets
Nach dem Trocknen werden 223 kg 9a) mit nachfolgender Beschichtung überzogen:

| | | |
|---|---|---|
| Eudragit E | (1) | 10,4kg |
| Triethylcitrat | (2) | 0,5 kg |
| Talkum | (3) | 6,0 kg |
| Magnesiumstearat | (4) | 1,2 kg |
| Ethanol | (5) | 75,0 kg |
| Wasser | (6) | 5,0 kg |

Entsprechend Beispiel 1b) wird die Suspension in bekannter Weise auf 223 kg Guaifenesin/Phenylephrin-Partikel aufgesprüht = 241,1 kg Guaifenesin/Phenylephrin-Pellets, speichelresistent.
9c) Herstellung der Guaifenesin/Phenylephrin-Pellets mit erfindungsgemäßer Beschichtung

| | | |
|---|---|---|
| Eudragit E | (1) | 10,0 kg |
| Carbopol 971 P | (2) | 11,0 kg |
| Talkum | (3) | 3,0 kg |
| Stearinsäure | (4) | 1,5 kg |
| Natriumhydrogencarbonat | (5) | 10,5 kg |
| Ethanol | (6) | 106,0 kg |

Entsprechend dem Beispiel 1d) wird die hergestellte Suspension auf 241,1 kg speichelresistente Guaifenesin/Phenylephrin-Pellets 9b) in bekannter Weise in einer Wirbelschicht-Apparatur aufgesprüht. Nach Trocknung erhält man 276,6 kg Pellets gemäß der Erfindung (12,8% erfindungsgemäße Beschichtung). Die erfindungsgemäßen Pellets dehnen sich in Wasser von 37°C innerhalb von 30 Sekunden unter einer Volumenzunahme von 36% aus und bilden eine weiche, glatte Pelletoberfläche. Die Pellets sind einzeln dispergiert und kleben nicht zusammen. Im Mund spürt man bereits nach 5 Sekunden eine weiche, glatte, mechanisch stabile Oberfläche, die durch Berührung mit der Zunge oder Drücken gegen den Gaumen nicht zerstört werden kann. Die Teilchen werden im Mund nicht als Fremdkörper empfunden und lassen sich leicht schlucken.
9d) Erfindungsgemäße Speichelfluss-fördernde Komponente

| | | |
|---|---|---|
| Sorbitol, 0,1 bis 1,0 mm | (1) | 558,0 kg |
| Natriumcarboxymethylcellulose | (2) | 10,0 kg |
| Aspartam | (3) | 2,0 kg |
| Tutti-Frutti Aroma (650916, Fa. Symrise) | (4) | 7,0 kg |
| Bananen-Aroma (214746, Fa. Symrise) | (5) | 2,0 kg |
| Magnesiumstearat | (6) | 3,0 kg |

werden durch ein Sieb der Maschenweite 1,5 mm gesiebt und 15 Minuten gemischt.
9e) Stick Pack-Abfüllung
276,6 kg der Guaifenesin/Phenylephrin-Pellets 9c) werden mit 1 kg Magnesiumstearat und 1,4 kg Talkum gemischt. Auf einer Abfüllmaschine werden mittels eines 2-Kammer Dosierschiebers in einem Arbeitsgang 279 mg dieser Mischung mit 582 mg der Speichelfluss-fördernden Komponente gemäß Beispiel 3 in ein Stick Pack gefüllt und verschlossen. Das Füllgewicht beträgt 861 mg und enthält 100 mg Guaifenesin und 5 mg Phenylephrin. Das Verhältnis der erfindungsgemäßen Guaifenesin/Phenylephrin-Pellets zur Speichelfluss-fördernden Mischung beträgt 1:2,1.
In gleicher Weise füllt man in einem Arbeitsgang 139,5 mg Pellets mit 291 mg der Speichelfluss-fördernden Komponente ab. Das Gesamtfüllgewicht beträgt 430,5 mg und enthält 50 mg Guaifenesin und 2,5 mg Phenylephrin.
9f) Im Dissolution-Test gemäß der Pharmakopoe, Paddle-Methode, 50 Umdrehungen/Minute im künstlichen Magensaft wird sowohl Guaifenesin als auch Phenylephrin innerhalb von 20 Minuten zu mehr als 90% freigesetzt. Die Pellets zeigen eine deutliche Tendenz zum Aufschwimmen.

### Beispiel 10

10a) Prednisolonphosphat-Natrium Pellets

| | | |
|---|---|---|
| Prednisolonphosphat-Natrium | (1) | 125 g |
| Povidone K25 | (2) | 100 g |
| Eudragit E | (3) | 500 g |
| Talkum | (4) | 100 g |
| Polyethylenglykol 6000 | (5) | 100 g |
| Ethanol | (6) | 4500 g |
| Wasser | (7) | 900 g |

In einer Mischung aus (6) und (7) werden die Komponenten (1), (2), (3) und (5) gelöst und die Komponente (4) suspendiert. Die Suspension wird auf 5000 g Non-Pareilles der Teilchengröße 0,5 bis 0,8 mm in einer Wirbelschicht-Apparatur gesprüht = 5925 g Prednisolonphosphat-Natrium Pellets.
10b) Prednisolonphosphat-Natrium Pellets mit erfindungsgemäßer Beschichtung

| | | |
|---|---|---|
| Eudragit E | (1) | 300 g |
| Carbopol 971 P | (2) | 200 g |
| Talkum | (3) | 140 g |
| Stearinsäure | (4) | 50 g |
| Natriumhydrogencarbonat | (5) | 180 g |
| Calciumcarbonat | (6) | 90 g |
| Ethanol | (7) | 3000 g |

In Ethanol werden (1) und (4) gelöst und die Komponenten (2), (3), (5) und (6) suspendiert. Die Suspension wird auf 5925 g Prednisolonphosphat-Natrium Pellets a) in bekannter Weise in einer Wirbelschicht-Apparatur aufgesprüht. Nach der Trocknung erhält man 6885 g Prednisolon-Pellets gemäß der Erfindung (13,9% erfindungsgemäßer Beschichtung, Wirkstoffgehalt 1,8%).
10c) Speichelfluss-fördernde Komponente

| | | |
|---|---|---|
| Mannit, sprühgetrocknet 0,05 bis 0,4 mm | (1) | 28000 g |
| Sorbit, 0,1 bis 1,0 mm | (2) | 11000 g |
| Natriumcarboxymethylcellulose | (3) | 700 g |
| Sucralose | (4) | 700 g |
| Weintrauben-Aroma (208275, Fa. Symrise) | (5) | 500 g |
| Himbeer-Aroma (652742, Fa. Symrise) | (6) | 500 g |
| Magnesiumstearat | (7) | 600 g |

10d) Entsprechend Beispiel 2 und 3 werden pro Stick Pack 139 mg (2,5 mg Prednisolonphosphat-Natrium) bzw. 278 mg (5 mg Prednisolonphosphat-Natrium) der Teilchen aus Beispiel 10b) mit 848 mg der Speichelfluss-fördernden Mischung aus Beispiel 10c) in ein Stick Pack gefüllt. Das Füllgewicht beträgt 987 bzw. 1126 mg. Das Verhältnis der erfindungsgemäßen Prednisolon-Pellets zur speichelfördernden Mischung beträgt 1:6,1 (2,5 mg Prednisolonphosphat-Natrium/Stick Pack) bzw. 1:3,05 (5 mg Prednisolonphosphat-Natrium/Stick Pack). Schüttet man den Inhalt eines Stick Packs auf die Zunge, so bildet sich innerhalb einer Minute eine Speichelmenge von durchschnittlich 2,4 ml. Die Viskosität des Speichels ist durch den Zusatz von Natriumcarboxymethylcellulose erhöht. In dem Speichel sind die erfindungsgemäßen Teilchen suspendiert. Die Oberfläche der Teilchen fühlt sich bei Berührung mit der Zunge weich und glatt an, durch Zungenbewegung oder Drücken gegen den Gaumen kann die erfindungsgemäße Oberfläche nicht entfernt werden.
Die erfindungsgemäßen Teilchen können leicht zusammen mit dem gebildeten Speichel praktisch vollständig geschluckt werden.

### Beispiel 11

11a) Speichelresistente Paracetamol-Teilchen

| | | |
|---|---|---|
| Paracetamol-Kristalle 0,05 bis 0,4 mm | (1) | 85,0 kg |
| Eudragit E | (2) | 10,0 kg |
| Talkum | (3) | 2,0 kg |
| Stearinsäure | (4) | 2,0 kg |
| Natriumlaurylsulfat | (5) | 1,0 kg |
| Wasser | (6) | 60,0 kg |

In einer Wirbelschicht-Apparatur werden Paracetamol-Kristalle in bekannter Weise mit einer wässrigen Eudragit E-Suspension entsprechend der Zusammensetzung 11a) überzogen und getrocknet. Man erhält 100,0 kg speichelresistente Paracetamol-Teilchen. Diese werden mit der erfindungsgemäßen Beschichtung entsprechend 11b) überzogen.
11b) Paracetamol-Teilchen mit erfindungsgemäßer Beschichtung

| | | |
|---|---|---|
| Eudragit E | (1) | 5,9 kg |
| Carbopol 971 P | (2) | 5,0 kg |
| Talkum | (3) | 2,5 kg |
| Stearinsäure | (4) | 1,0 kg |
| Natriumhydrogencarbonat | (5) | 2,5 kg |
| Ethanol | (6) | 50,0 kg |

Nach der Trocknung erhält man 116,9 kg Paracetamol-Teilchen mit der erfindungsgemäßen Beschichtung. Die Teilchen werden mit 1,1 kg Magnesiumstearat gemischt. Der Wirkstoffgehalt beträgt 72,0%.
11 c) Speichelfluss-fördernde Mischung

| | | |
|---|---|---|
| Sorbit, 0,1 bis 1,0 mm | (1) | 100,0 kg |
| Natriumcarboxymethylcellulose | (2) | 2,5 kg |
| Cappuccino-Aroma (142618, Fa. Symrise) | (3) | 1,5 kg |
| Speichelfluss-förderndes Aroma | | |
| (Optaflow, 150226, Fa. Symrise) | (4) | 0,9 kg |
| Sucralose | (5) | 0,5 kg |
| Magnesiumstearat | (6) | 1,5 kg |

11d) 250, 500 oder 1000 mg Paracetamol entsprechende Mengen an erfindungsgemäß überzogenen Paracetamol-Teilchen werden wie in der nachfolgenden Tabelle angegeben mit der Speichelfluss-fördernden Komponente gemischt und in Stick Packs abgefüllt. Im Falle der Stick Packs mit 1000 mg Paracetamol wird der Anteil an Speichelfluss-förderndem Aroma (Optaflow) entsprechend der Zusammensetzung 11c) von 0,9 kg auf 1,8 kg erhöht.

| Paracetamol | Paracetamol-Teilchen entsprechend der Erfindung A | Speichelfluss-fördernde Komponente B | Verhältnis A:B |
|---|---|---|---|
| 250 mg | 347 mg | 300 | 1,16:1 |
| 500 mg | 694 mg | 500 | 1,39:1 |
| 1000 mg | 1388 mg | 600 | 2,3:1 |

Schüttet man den Inhalt eines Stick Packs auf die Zunge, so löst sich die Speichelfluss-fördernde Komponente in ca. 30 Sekunden. Die ursprünglich sandigen, scharfkantigen Paracetamol-Kristalle bilden innerhalb von 30 Sekunden mit der erfindungsgemäßen Beschichtung eine angenehme, weiche, aber mechanisch stabile Oberfläche. Die Teilchen lassen sich mit dem gebildeten Speichel leicht schlucken.

### Beispiel 12

12a) Acetylsalicylsäure-Teilchen mit erfindungsgemäßer Beschichtung

| | | |
|---|---|---|
| Acetylsalicylsäure-Teilchen 0,05 bis 0,4 mm | (1) | 100,0 kg |
| Eudragit E | (2) | 6,0 kg |
| Carbopol 971 | (3) | 4,5 kg |
| Stearinsäure | (4) | 1,5 kg |
| Natriumhydrogencarbonat | (5) | 2,0 kg |
| Mononatriumcitrat, mittlere Korngröße 25 µm | (6) | 1,0 kg |
| Ethanol | (7) | 85,0 kg |

In bekannter Weise wird aus den Komponenten eine ethanolische Eudragit E-Suspension hergestellt, die in einer Wirbelschicht-Apparatur auf Acetylsalicylsäure-Teilchen aufgesprüht wird. Auf 100 kg Acetylsalicylsäure-Teilchen werden 15 kg erfindungsgemäßer Beschichtung aufgetragen. Die erfindungsgemäßen Teilchen werden sorgfältig getrocknet.
Der Wirkstoffgehalt der Teilchen beträgt 87%.
12b) Acetylsalicylsäure/Paracetamol/Koffein-Stick Packs 250/250/62,5 mg
In bekannter Weise wird eine Mischung hergestellt aus 287,4 mg Acetylsalicylsäure-Teilchen gemäß Beispiel 12a), 347 mg Paracetamol-Teilchen gemäß Beispiel 11b), 87 mg Koffein-Teilchen gemäß Beispiel 7b) und 400 mg Speichelfluss-fördernde Komponente gemäß Beispiel 11c) und in ein Stick Pack abgefüllt. Die Analgetika-Kombination wird aus dem Stick Pack direkt auf die Zunge geschüttet und kann nach 30 Sekunden mit dem gebildeten Speichel bequem geschluckt werden. Alle Teilchen haben eine weiche, im Mund als angenehm empfundene Teilchen-Oberfläche und können bequem ohne Wasser geschluckt werden.
Zur Behandlung von Migräne werden in gleicher Weise Stick Packs hergestellt, die 500 mg Acetylsalicylsäure, 500 mg Paracetamol und 130 mg Koffein mit der erfindungsgemäßen Beschichtung enthalten. Die Speichelfluss-fördernde Komponente 11c) beträgt 500 mg. Zur Erhöhung des Speichelflusses enthält sie aber die doppelte Menge Speichelfluss-förderndes Aroma (Optaflow, Fa. Symrise).

## Patentansprüche

1. Pharmazeutische Zusammensetzung zur oralen Verabreichung in Form von zumindest einem wirkstoffhaltigen Teilchen, wobei das zumindest eine wirkstoffhaltige Teilchen umfasst
a) einen Kern, der den Wirkstoff des wirkstoffhaltigen Teilchens und gegebenenfalls geeignete Hilfsstoffe enthält und
b) eine oder mehrere Beschichtungen, die auf dem Kern aufgebracht sind, wobei die eine Beschichtung oder die äußerste der mehreren Beschichtungen
(i) mindestens ein mit Wasser gelbildendes Polymer,
(ii) mindestens ein mit Wasser nicht-gelbitdendes Polymer,
(iii) mindestens eine Verbindung, die Kohlendioxid freisetzen kann, sowie
(iv) gegebenenfalls weitere Hilfsstoffe enthält.

2. Pharmazeutische Zusammensetzung nach Anspruch 1, **dadurch gekennzeichnet, dass** das Polymer gemäß b)(ii) ein im Magensaft lösliches Polymer ist.

3. Pharmazeutische Zusammensetzung nach Anspruch 1 oder 2, **dadurch gekennzeichnet, dass** das Polymer gemäß b)(ii) ein Copolymerisat mit kationischem Charakter auf Basis von Dimethylaminomethylmethacrylat und neutralen Methacrylsäureestern ist.

4. Pharmazeutische Zusammensetzung nach einem der Ansprüche 1 bis 3, **dadurch gekennzeichnet, dass** das Polymer gemäß b)(i) ein Polymer ist, das in einer Menge von 2% in Wasser bei einem pH-Wert von 5,0 oder weniger löslich ist oder eine Dispersion bildet, die eine Viskosität von 100 mPa·s oder weniger aufweist.

5. Pharmazeutische Zusammensetzung nach einem der Ansprüche 1 bis 4, **dadurch gekennzeichnet, dass** das Polymer gemäß b)(i) quervernetzte Polyacrylsäure oder ein quervernetztes Polyacrylat ist.

6. Pharmazeutische Zusammensetzung nach einem der Ansprüche 1 bis 5, **dadurch gekennzeichnet, dass** das Polymer gemäß b)(i) ein Carbomer ist.

7. Pharmazeutische Zusammensetzung nach einem der Ansprüche 1 bis 6, **dadurch gekennzeichnet, dass** die Verbindung gemäß b)(iii), die Kohlendioxid freisetzen kann, entweder eine Verbindung der Formel MHₙ(CO₃) darstellt, wobei M ein Alkali- oder Erdalkalimetallion darstellt und n = 0 oder 1 ist, wobei n = 0 ist, wenn M ein Erdalkalimetallion ist, oder Natriumglycincarbonat ist.

8. Pharmazeutische Zusammensetzung nach einem der Ansprüche 1 bis 7, **dadurch gekennzeichnet, dass** zumindest einer der Bestandteile b)(i) und b)(ii) ein saures Polymer ist.

9. Pharmazeutische Zusammensetzung nach einem der Ansprüche 1 bis 8, **dadurch gekennzeichnet, dass** die eine Beschichtung oder die äußerste der mehreren Beschichtungen weiterhin eine Säure enthält.

10. Pharmazeutische Zusammensetzung nach Anspruch 9, **dadurch gekennzeichnet, dass** die Säure ausgewählt ist aus Zitronensäure, Weinsäure, Fumarsäure, Adipinsäure, Mononatriumhydrogencitrat, Mononatriumtartrat, Mononatriumphosphat und Gemischen davon.

11. Pharmazeutische Zusammensetzung nach einem der Ansprüche 1 bis 9, **dadurch gekennzeichnet, dass** der Wirkstoff ausgewählt ist aus Guaifenesin, Prednisolon, Dextromethorphan oder einem pharmazeutisch verträglichen Salz davon, einem Gemisch aus Guaifenesin und Dextromethorphan oder einem pharmazeutisch verträglichen Salz davon und einem Gemisch aus Guaifenesin und Phenylephrin oder einem pharmazeutisch verträglichen Salz davon.

12. Pharmazeutische Zusammensetzung nach einem der Ansprüche 1 bis 11, **dadurch gekennzeichnet, dass** das Verhältnis zwischen den Polymeren b)(ii) und b)(i) im Bereich von 10:1 bis 1:5 liegt.

13. Pharmazeutische Zusammensetzung nach einem der Ansprüche 1 bis 12, **dadurch gekennzeichnet, dass** das Verhältnis zwischen dem Polymer b)(i) und der Kohlendioxid-freisetzenden Verbindung b)(iii) im Bereich von 10:1 bis 1:5 liegt.

14. Arzneimittel, umfassend eine pharmazeutische Zusammensetzung nach einem der Ansprüche 1 bis 13 und mindestens eine den Speichelfluss fördernde Verbindung, die nicht Bestandteil der pharmazeutischen Zusammensetzung ist.

15. Arzneimittel nach Anspruch 14, **dadurch gekennzeichnet, dass** die Speichelfluss-fördernde Verbindung ausgewählt ist aus Saccharose, Glucose, Maltodextrin, Sorbit, Mannit, Xylit, Maltit, Malbit, Inulin, Isomalt, Aromen und Gemischen davon.

16. Arzneimittel nach Anspruch 14 oder 15, **dadurch gekennzeichnet, dass** die pharmazeutische Zusammensetzung nach einem der Ansprüche 1 bis 12 im Gemisch mit der Speichelfluss-fördernden Verbindung vorliegt.

17. Arzneimittelpackung, umfassend eine pharmazeutische Zusammensetzung gemäß einem der Ansprüche 1 bis 13, gegebenenfalls mindestens eine den Speichelfluss fördernde Verbindung und eine Anweisung, die pharmazeutische Zusammensetzung und die Speichelfluss-fördernde Verbindung gemeinsam zu verabreichen.

18. Verwendung einer pharmazeutischen Zusammensetzung nach einem der Ansprüche 1 bis 13 zur Herstellung eines Arzneimittels zur direkten oralen Verabreichung an einen Patienten ohne vorheriges Inkontaktbringen des Arzneimittels mit einer Flüssigkeit.

19. Verwendung nach Anspruch 18, wobei die Zusammensetzung nach einem der Ansprüche 1 bis 13 zur gleichzeitigen Verabreichung mit mindestens einer den Speichelfluss fördernden Verbindung vorgesehen ist.

20. Verwendung einer pharmazeutischen Zusammensetzung nach einem der Ansprüche 1 bis 13 zur Herstellung eines Arzneimittels in Form einer zum Trinken geeigneten wässrigen Dispersion der Teilchen.
